(19) Europäisches Patentamt
European Patent Office
Office européen des brevets

(11) Publication number: **0 200 736 B1**

(12) **EUROPEAN PATENT SPECIFICATION published in accordance with Art. 158(3) EPC**

(45) Date of publication of patent specification: **24.04.91**

(51) Int. Cl.[5]: **C07D 405/06**, C07D 231/12, C07D 231/14

(21) Application number: **85903248.4**

(22) Date of filing: **18.06.85**

(86) International application number: **PCT/EP85/00293**

(87) International publication number: **WO 86/00307 (16.01.86 86/02)**

(54) **PYRAZOLE ANALOGS OF MEVALONOLACTONE AND DERIVATIVES THEREOF, PROCESSES FOR THEIR PRODUCTION AND THEIR USE.**

(30) Priority: **22.06.84 US 623393**

(43) Date of publication of application: **12.11.86 Bulletin 86/46**

(45) Publication of the grant of the patent: **24.04.91 Bulletin 91/17**

(84) Designated Contracting States: **AT BE CH DE FR GB IT LI LU NL SE**

(56) References cited:
**EP-A- 0 019 760**
**US-A- 3 254 093**
**US-A- 4 245 106**

**Tetrahedron Letters 23(1982), 281-284**

(73) Proprietor: **SANDOZ AG**
**Lichtstrasse 35**
**CH-4002 Basel(CH)BE CH FR GB IT LI LU NL SE**

Proprietor: **SANDOZ-PATENT-GMBH**
**Humboldtstrasse 3**
**W-7850 Lörrach(DE)DE**

Proprietor: **SANDOZ-ERFINDUNGEN Verwaltungsgesellschaft m.b.H.**
**Brunner Strasse 59**
**A-1235 Wien(AT)AT**

(72) Inventor: **WAREING, James, Richard**
**402 Millbrook Avenue**
**Randolph, NJ 07801(US)**

EP 0 200 736 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

**Description**

The invention concerns pyrazole analogs of mevalonolactone and deivatives thereof, processes for their production, pharmaceutical compositions containing them and their use as pharmaceuticals in particular as hypolipoproteinemic and anti-atherosclerotic agents.

The invention is concerned with compounds of formula I

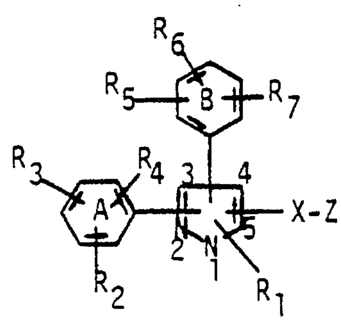

I

wherein $R_1$ is $C_{1-6}$alkyl,
each of $R_2$ and $R_5$ is independently hydrogen,$C_{1-4}$alkyl, $C_{1-4}$alkoxy (except t-butoxy), trifluoromethyl, fluoro, chloro, phenyl, phenoxy or benzyloxy,
each of $R_3$ and $R_6$ is independently hydrogen, $C_{1-3}$alkyl, $C_{1-3}$alkoxy, trifluoromethyl, fluoro, chloro, phenoxy or benzyloxy,
each of $R_4$ and $R_7$ is independently hydrogen, $C_{1-2}$alkyl, $C_{1-2}$alkoxy, fluoro or chloro,
with the proviso that there may only be one each of trifluoromethyl, phenoxy or benzyloxy on each of rings A and B,
X is $-(CH_2)_2-$ or -CH = CH-

and Z is 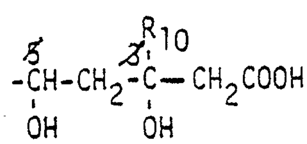 II

wherein $R_{10}$ is hydrogen or $C_{1-3}$alkyl, whereby either the -X-Z group is in the 4-position of the pyrazole ring and $R_1$ is in the 3- or 5-position, or the -X-Z group is in the 5-position and $R_1$ is in the 1- or 4-position; in free acid form or in the form of a physiologically acceptable ester thereof or in the form of a δ-lactone thereof wherein Z has the formula IIb

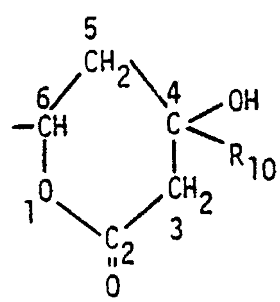

IIb

or in salt form.

Physiologically acceptable include physiologically hydrolysable- und -acceptable esters. By the term "physiologically-hydrolysable and -acceptable ester" is meant an ester of a compound in accordance with the invention in which the carboxyl moiety if present is esterified, and which is hydrolysable under physiological conditions to yield an alcohol which is itself physiologically acceptable, e.g. non-toxic at desired dosage levels. Preferred such esters as Z can be represented together with the free acid by formula IIa

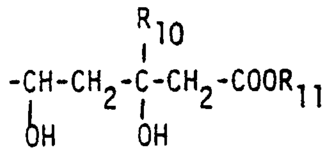

IIa

wherein $R_{11}$ is hydrogen, $C_{1-4}$alkyl or benzyl preferably hydrogen, $C_{1-3}$alkyl, n-butyl, i-butyl, t-butyl or benzyl
and $R_{10}$ is as defined above.

When IIa is in salt form $R_{11}$ represents a cation.

References to "lactone" hereinafter refer to δ-lactones.

Salts of the compounds of the invention, e.g. of the compounds of formula I, include in particular their pharmaceutically acceptable salts. Such pharmaceutically aceptable salts include e.g. alkali metal salts such as the sodium and potassium salts and salts with ammonium.

References to compounds of formula I, II, IIa and IIb and sub-species thereof are intended to cover all forms unless otherwise stated.

Depending on the position of the various substituents in the pyrazole ring compounds of formula I may divided into four groups as follows:

| Compound | Position in pyrazole ring | | | |
|---|---|---|---|---|
| Group | 1 | 3 | 4 | 5 |
| Formula IA | $R_1$ | ring A | ring B | -X-Z |
| Formula IB | ring B | ring A | $R_1$ | -X-Z |
| Formula IC | ring A | ring B | -X-Z | $R_1$ |
| Formula ID | ring A | $R_1$ | -X-Z | ring B |

These four groups may be further divided into two sub-groups each depending on the significance of Z as either a group of formula II in other than lactone form (sub-group "a") or a group of formula IIb (sub-group "b"). There resulting eight sub-groups are designated as formulae IAa, IAb, IBa, IBb, ICa, ICb, IDa, IDb respectively.

As is self-evident to those skilled in the art, each compound of formula I (and every sub-group and species thereof) has at least two centres of asymmetry (e.g. the two carbon atoms bearing the hydroxy groups in the group of formula IIa and the carbon atom bearing the hydroxy group and the carbon atom having the free valence in the group of formula IIb) and these lead (e.g. with two centres) to four stereoisomeric forms (enantiomers) of each compound (two racemates or pairs of diastereoisomers). In the preferred compounds having only two such centres of asymmetry these four stereoisomers may be designated as the R,R; R,S; S,R; and S,S enantiomers, all four stereoisomers being within the scope of this invention. Depending on the nature of substituents further asymmetric carbon atoms may be present and the resulting isomers and mixtures thereof also form part of the invention. Compounds containing only two centres of asymmetry (four mentioned stereoisomers) are preferred.

$R_1$ preferably does not contain an asymmetric carbon atom and is preferably $R_1'$, where $R_1'$ is $C_{1-3}$alkyl, n-butyl or i-butyl, more preferably $R_1''$, where $R_1''$ is $C_{1-3}$alkyl and most preferably isopropyl.

$R_2$ is preferably hydrogen, $C_{1-3}$alkyl, n-butyl, sec.-butyl, t-butyl $C_{1-3}$alkoxy, n-butoxy, sec.-butoxy, trifluoromethyl, fluoro, chloro, phenyl, phenoxy or benzyloxy and in particular $R_2'$ where $R_2'$ is hydrogen, $C_{1-3}$alkyl,
$C_{1-3}$alkoxy, trifluoromethyl, fluoro or chloro, more preferably $R_2''$, where $R_2''$ is hydrogen or fluoro, and most preferably hydrogen.

$R_3$ is preferably $R_3'$, where $R_3'$ is hydrogen, $C_{1-2}$alkyl, $C_{1-2}$alkoxy, fluoro or chloro, and most preferably hydrogen.

$R_4$ is preferably $R_4'$, where $R_4'$ is hydrogen or methyl, and most preferably hydrogen.

The $R_2$-bearing phenyl group (ring A) is preferably unsubstituted.

$R_5$ is preferably hydrogen, $C_{1-3}$alkyl, n-butyl, sec.-butyl, t-butyl, $C_{1-3}$alkoxy, n-butoxy, sec.-butoxy,

trifluoromethyl, fluoro, chloro, phenyl, phenoxy or benzyloxy and in particular $R_5'$, where $R_5'$ is hydrogen, $C_{1-3}$alkyl, $C_{1-3}$alkoxy, trifluoromethyl, fluoro or chloro, more preferably $R_5'$, where $R_5''$ is hydrogen or fluoro, and most preferably fluoro.

$R_6$ is preferably $R_6'$, where $R_6'$ is hydrogen, $C_{1-2}$alkyl, $C_{1-2}$alkoxy, fluoro or chloro, more preferably $R_6''$, where $R_6''$ is hydrogen or methyl, and most preferably hydrogen.

$R_7$ is preferably $R_7'$, where $R_7'$ is hydrogen or methyl, and most preferably hydrogen.

Preferably, when two of $R_5$ ($R_5'$, etc.), $R_6$ ($R_6'$, etc.) and $R_7$ ($R_7'$ etc.) are other than hydrogen and one is hydrogen, at least one of the two that are other than hydrogen is in a meta or para position and not more than one of them is a member of the group consisting of t -butyl, trifluoromethyl, phenyl, phenoxy and benzyloxy; more preferably, the two that are other than hydrogen are not ortho to each other when neither of them is a member of the group consisting of methyl, methoxy, fluoro and chloro.

Preferably, when each of $R_5$ ($R_5'$, etc.), $R_6$ ($R_6'$, etc.) and $R_7$ ($R_7'$ etc.) is other than hydrogen, at least two of them are in meta or para positions, and not more than one of them is a member of the group consisting of t -butyl, trifluoromethyl, phenyl, phenoxy and benzyloxy; more preferably, no two of them are ortho to each other unless at least one member of each pair of substituents that are ortho to each other is a member of the group consisting of methyl, methoxy, fluoro and chloro.

The $R_5$-bearing phenyl group (ring B) is preferably 4-fluorophenyl or 3,5-dimethylphenyl, preferably the former.

$R_{10}$ is preferably $R_{10}'$, where $R_{10}'$ is hydrogen or methyl, and most preferably hydrogen.

$R_{11}$ is preferably $R_{11}'$, where $R_{11}'$ is hydrogen or $C_{1-3}$alkyl, more preferably $R_{11}''$ which is hydrogen or $C_{1-2}$alkyl.

Compounds of formula I wherein Z is of formula II or IIa are most preferably in salt form. Preferred salt-forming cations are those free from centres of asymmetry especially e.g. sodium, potassium or ammonium, most preferably sodium. Such cations may also be di- or tri-valent and are balanced by 2 or 3 carboxylate containing anions. Any -CH=CH- containing bridge as X is preferably trans i.e. (E).

X is $-CH_2CH_2-$ or -CH=CH-, more preferably -CH=CH- and most preferably

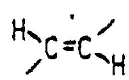

(i.e. (E)-CH=CH-).

Z is preferably a group of formula IIa wherein $R_{10}$ is $R_{10}'$ (especially hydrogen) or a group of formula IIb, more preferably a group of formula IIa, wherein $R_{10}$ is hydrogen and $R_{11}$ is $R_{11}'$ or a cation or a group of formula IIb, even more preferably a group of formula IIa wherein $R_{10}$ is hydrogen, and $R_{11}$ is $R_{11}''$ or a cation; or a group of formula IIb, and most preferably a group of formula IIa wherein $R_{10}$ is hydrogen, and $R_{11}$ is a cation, especially sodium.

As between otherwise identical compounds of formula I, those wherein Z is in other than lactone form are generally preferred over those wherein Z is a group of formula IIb.

Insofar as the compounds of Groups IAa, IBa, ICa and IDa and each of the sub-groups thereof are concerned, the erythro isomers are preferred over the threo isomers, erythro and threo referring to the relative positions of the hydroxy groups in the 3- and 5-positions of the group of formula II.

Insofar as the compounds of Groups IAb, IBb, ICb and ICb and each of the sub-groups thereof are concerned, the trans lactones are generally preferred over the cis lactones, cis and trans referring to the relative positions of $R_{10}$ and the hydrogen atom in the 6-position of the group of formula IIb.

The preferred stereoisomers of the compounds of formula I having only two centres of asymmetry wherein X is, -CH=CH-, and Z is a group of formula II, are the 3R,5S isomer and the racemate or which it is a constituent, i.e. , the 3R,5S-3S,5R (erythro) racemate.

The preferred stereoisomers of the compounds of formula I having only two centres of asymmetry wherein X is, $-CH_2CH_2-$, , and Z is a group of formula II, are the 3R,5R isomer and the racemate of which it is a constituent, i.e., the 3R,5R-3S,5S (erythro) racemate.

The preferances set forth in the preceding two paragraphs also apply to the compounds of formula I having more than two centres of asymmetry and represent the preferred configurations of the indicated positions.

The preferred stereoisomers of the compounds of formula I wherein X, -CH=CH-, and Z is a group of formula IIb are the 4R,6S and 4R,6R isomers and the racemate of which each is a constituent, i.e., the 4R,6S-4S,6R (trans lactone) and 4R,6R-4S,6S (cis lactone) racemates with the 4R,6S isomer and the racemate of which it is a constituent being more preferred and the 4R,6S isomer being most preferred.

The preferred stereoisomers of the compounds of formula I wherein X is, $-CH_2CH_2-$, , and Z is a group

of formula IIb are the 4R,6R and 4R,6S isomers and the racemate of which each is a constituent, i.e., the 4R,6R-4S,6S (trans lactone) and 4R,6S-4S,6R (cis lactone) racemates, with the 4R,6R isomer and the racemate of which it is a constituent being more preferred and the 4R,6R isomer being most preferred.

Each of the preferences set for the above applies, not only to the compounds of formula I, but also to the compounds of groups IA, IB, IC and ID and those of Groups IAa, IAb, IBa, IBb, ICa, ICb, IDa and IDb as well as to every other sub-group thereof set forth in the specification, e.g., Groups (i) et.seq., unless otherwise indicated. When any preferance or group contains a variable, the preferred significances of that variable apply to the preference in question, unless otherwise indicated.

Preferred groups of compounds of formula IAa and IAb include the compounds

(i) of Group IAa wherein $R_1$ is $R_1'$, $R_2$ is $R_2'$, $R_3$ is $R_3'$, $R_4$ is $R_4'$, $R_5$ is $R_5'$,$R_6$ is $R_6'$, $R_7$ is $R_7'$ and $R_{10}$ is $R_{10}'$, ,

(ii) of (i) wherein $R_2$ is $R_2''$, $R_3$ is hydrogen, $R_4$ is hydrogen, $R_5$ is $R_5''$, $R_6$ is $R_6''$, $R_{10}$ is hydrogen, $R_{11}$ is $R_{11}'$ or a cation and X is (E)-CH=CH-,

(iii) of (ii) wherein $R_1$ is $R_1''$,

(iv)-(vi) of (i)-(iii) wherein $R_{11}$ is a cation, especially sodium,

(vii)-(xii) of (i)-(vi) wherein the hydroxy groups in the 3- and 5-positions of the group of formula IIa have the erythro configuration.

(xiii)-(xviii) the 3R,5S enantiomers of the compounds of (vii)-(xii) wherein X is -CH=CH- and the 3R,5R enantiomers of those wherein X is - $CH_2CH_2$-.

(xiii)-(xviii) the 3R,5S enantiomers of the compounds of (vii)-(xii),

(xix) of Group IAb wherein $R_1$ is $R_1'$, $R_2$ is $R_2'$, $R_3$ is $R_3'$, $R_4$ is $R_4'$, $R_5$ is $R_5'$, $R_6$ is $R_6'$, $R_7$ is $R_7'$ and $R_{10}$ is $R_{10}'$.

xx) of (xix) wherein $R_2$ is $R_2''$, $R_3$ is hydrogen, $R_4$ is hydrogen, $R_5$ is $R_5''$, $R_6$ is $R_6''$, $R_{10}$ is hydrogen, and X is (E)-CH=CH-,

(xxi) of (xx) wherein $R_1$ is $R_1''$,

(xxii)-(xxiv) of (xix)-(xxi) wherein $R_{10}$ and the hydrogen atom in the 6-position of the group of formula IIb are trans to each other

A particular compound group covers those compounds of formula I wherein

$R_1$ is $C_{1-6}$alkyl not containing an asymmetric carbon atom,

each of $R_2$ and $R_5$ is independently hydrogen, $C_{1-3}$alkyl, n-butyl, i-butyl, t-butyl, $C_{1-3}$alkoxy, n-butoxy, sec-butoxy, trifluoromethyl, fluoro, chloro, phenyl, phenoxy or benzyloxy,

each of $R_3$ and $R_6$ is independently hydrogen,$C_{1-3}$alkl, $C_{1-3}$alkoxy, trifluoromethyl, fluoro, chloro, phenoxy or benzyloxy,

each of $R_4$ and $R_7$ is independently hydrogen, $C_{1-2}$alkyl, $C_{1-2}$alkoxy, fluoro or chloro,

with the provisos that not more than one of $R_2$ and $R_3$ is trifluoromethyl, not more than one of $R_2$ and $R_3$ is phenoxy, not more than one of $R_2$ and $R_3$ is benzyloxy, not more than one of $R_5$ and $R_6$ is trifluoromethyl, not more than one of $R_5$ and $R_6$ is phenoxy, and not more than one of $R_5$ and $R_6$ is benzyloxy,

X is -$CH_2CH_2$- or -CH=CH-, and

Z is 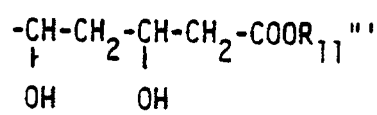

or 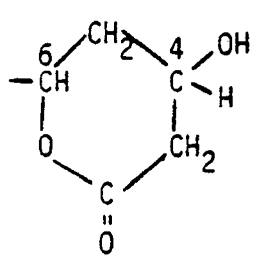 ,

wherein $R_{11}'''$ is hydrogen, $R_{12}$ or M,

wherein $R_{12}$ is a physiologically acceptable and hydrolyzable ester group, and

M is a pharmaceutically acceptable cation.

whereby either the -X-Z group is in the position of the pyrazole ring, and $R_1$ is in the 3- or 5-position or the -X-Z group is in the 5-position and $R_1$ is in the 1- or 4-position

The compounds of formula I can be prepared by the following reactions wherein Py stands

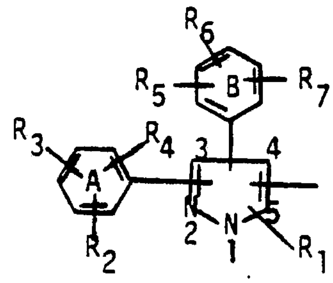

for
wherein $R_1$ to $R_7$ are as defined above, including the provisos

a) when $R_{10}$ is hydrogen reducing a compound of formula XX

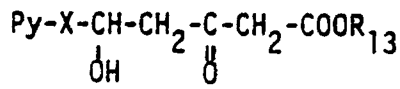

XX

wherein $R_{13}$ is a radical forming an ester
and X is as defined above,

b) when $R_{10}$ is $C_{1-3}$alkyl hydrolysing a compound of formula XVIIE

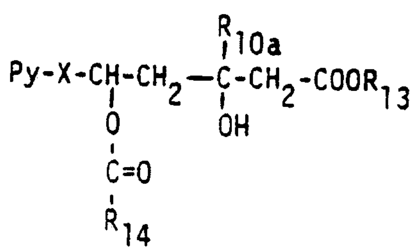

XVIIE

wherein $R_{10a}$ is $C_{1-3}$alkyl, $R_{14}$ is part of an ester forming group
and X and $R_{13}$ are as defined above,

c) deprotecting a compound of formula V

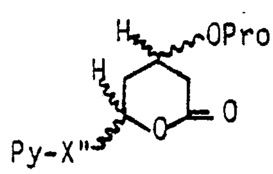

V

wherein X" represents $-CH_2CH_2$ or $-CH=CH-$ and Pro is a protecting group

d) deprotecting
a compound of formula XI

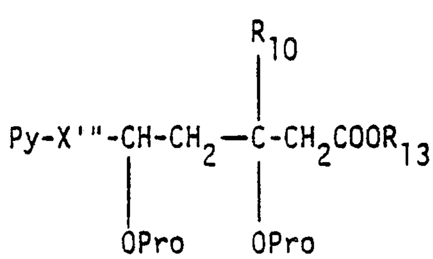

XI

wherein X'" is $-(CH_2)_2-$, or $-CH=CH-$
and q, $R_{10}$, $R_{13}$ and Pro are as defined above,

e) hydrolysing a compound of formula I in the form of an ester or a lactone or

f) esterifying or lactonising a compound of formula I in free acid form,

and when a free carboxyl group is present, recovering the compound obtained in free acid form or in the form of a salt. In processes a), b) and d) $R_{13}$ is preferably $C_{1-3}$alkyl, n-butyl, i-butyl, t-butyl or benzyl, more preferably $C_{1-3}$alkyl and especially $C_{1-2}$alkyl and $R_{14}$ is preferably $C_{1-3}$alkyl more preferably n-$C_{1-3}$alkyl, especially $C_{1-2}$alkyl.

Process a) is particularly suited for compounds wherein X is $-(CH_2)_2-$ or (E)-CH=CH- and in ester form.

Process b) is particularly suited for compounds wherein X is $-(CH_2)_2-$ or (E)-CH=CH- in salt form.

Process c) is particularly suited for compounds wherein X is -CH=CH- and the lactone is in 4R,6S configuration and those wherein X is $-CH_2-CH_2-$ and the lactone is in 4R,6R configuration.

Process d) is particularly suited for compounds in ester form.

It will readily be appreciated that the various forms of the compounds of formula I may be interconverted as indicated in e) and f) above.

In the same way compounds obtained according to a), b), c) and d) may be hydrolysed to free acid forms and free acid forms may be esterified or lactonised to produce a desired end-product. The invention thus also provides a process for preparing a compound of formula I which comprises hydrolysing a compound of formula I in ester or lactone form or esterifying or lactonising a compound of formula I in free acid form and when a free carboxyl group is present recovering the compound obtained in free acid form or in the form of a salt.

Unless otherwise stated rections are performed in a manner conventional for the type of reaction involved. Molar ratios and reaction times are as a rule conventional and non-critical and are chosen according to principles well established in the art on the basis of reactions and conditions employed.

Solvents, alone or as mixtures, are generally chosen which remain inert and liquid during the reaction in question.

Examples of inert atmospheres are carbon dioxide and more usually nitrogen or a nobel gas, nitrogen being preferred. Most reactions, including those wherein use of an inert atmosphere is not mentioned, are carried out in such for convenience.

EP-A-114027 and 117228 including the examples thereof disclose analogous processes and further suitable reaction conditions.

Relevant prior art includes 3b) J.D. Prugh and A.A. Deana, Tetr. Lett. 23 (1982) 281-284. This reference discloses inhibitors of HMG-CoA reductase, in particular the chemical structure of Mevinolin and Compactin.

Reduction according to a) is preferably carried out using a mild reducing agent such as sodium borohydride or, a complex of t-butylamine and borane in an inert organic solvent such as a lower alkanol, preferably ethanol, conveniently at a temperature of -10° to 30° C, under an inert atmosphere.

Use of an optically pure starting material will lead to only two optical isomers (diastereoisomers) of the resulting end product. However, if stereospecificity is desired it is preferred to utilize a stereo-selective reduction in order to maximize production of a mixture of the erythro stereoisomers (racemate) of which the preferred stereoisomer (as set forth above) it a constituent. Stereoselective reduction is preferably carried out in three steps. For example in the first step, the ketoester of formula XX is treated with a tri(primary or secondary $C_{2-4}$alkyl)borane, preferably triethylborane or tri-n-butylborane, and optionally air to form a complex. The reaction temperature is suitably 0° to 50° C, preferably 0° to 25° C. The first step is carried out in an anhydrous inert organic solvent, preferably an ether solvent such as tetrahydrofuran, diethyl ether, 1,2-dimethoxyethane or 1,2-diethoxyethane, with tetrahydrofuran, being the most preferred solvent. In the second step, the complex is reduced with sodium borohydride, preferably in the same solvent as utilized for the first step, at -100° to -40° C, preferably -90° to -70° C. In the third step, the product of the second step is, for example, treated with, preferably, anhydrous methanol at 20° to 60° C, preferably 20° to 30° C. The amount of methanol is not critical. However, a large excess, e.g. , 50-500 moles per mole of ketoester of formula XX, is typically utilized.

Hydrolysis according to b) or e) is carried out in a manner conventional for such reactions e.g. employing an inorganic hydroxide such as NaOH or KOH with, if desired subsequent acidification to give the free acid form. Suitable solvents are mixtures of water and water miscible solvents such as lower alkanols e.g. methanol or ethanol and reaction conveniently takes place at temperatures from 0° C to reflux preferably 0° to 75° C e.g. 20 to 70° C. If it is desired to recover the compound in a salt form corresponding to the cation of the hydroxide employed then slightly less than equivalent amounts of the latter may be employed. In b) $R_{14}$ will conveniently be the same as $R_{13}$ e.g. $C_{1-3}$alkyl more preferably n-$C_{1-3}$alkyl, especially $C_{1-2}$.

Lactonisation according to f) is carried out in conventional manner e.g. by heating the corresponding acid in an anhdrous inert organic solvent e.g. a hydrocarbon such as benzene, toluene or a xylene or

mixtures thereof, preferably at temperatures of 75° C to reflux although more preferably not above 150° C. Preferably, however, a lactonisation agent, e.g. a carbodiimide, preferably a water-soluble carbodiimide such as N-cyclohexyl-N'-[2'-(N"-methylmorpholinium)ethyl]carbodiimide p-toluenesulfonate, in an anhydrous inert organic solvent, e.g. a halogenated lower alkane, preferably methylene chloride is employed. Reaction temperatures then lie typically between 10° and 35° C, especially 20° to 25°.

As is evident to those in the art, a racemic threo 3,5-di-hydroxycarboxylic acid yields a racemic cis lactone (two stereoisomers) and a racemic erythro 3,5-dihydroxycarboxylic acid yields a racemic trans lactone (two stereoisomers). Likewise if a single enantiomer of the 3,5-dihydroxycarboxylic acid is utilized, a single enantiomer of the lactone is obtained. For example, lactonisation of a 3R,5S erythro dihydroxycarboxylic acid yields a 4R,6S lactone.

Esterification according to f) is conventional, employing e.g. a large excess of a compound $R_{13}OH$, wherein $R_{13}$ is as defined above at 0-70° C, e.g. 20° C to 40° C in the presence of a catalytic amount of an acid such as p-toluenesulfonic acid. Where methyl esters are required these can also be obtained e.g. using diazomethane in an anhydrous inert ether solvent such as tetrahydrofuran, 1,2-dimethoxyethane or 1,2-di-ethoxyethane and especially diethylether at e.g. 0° to 30° C preferably 20° to 30° C.

Preferably, however, esterification takes place by first forming the corresponding lactone and reacting this with $M_2^{\oplus}$ $^{\ominus}OR_{13}$ ($M_2^{\oplus}$ = $Na^{\oplus}$ or $K^{\oplus}$) at 0° to 70° C preferably 20° to 25° C in an inert organic solvent e.g. an ether such as tetrahydrofuran or an alcohol of formula $R_{13}OH$ if a liquid.

Examples of protecting groups in reaction c) and d) are diphenyl-t-butylsilyl, tri-isopropylsilyl or dimethyl-t-butylsilyl, benzyl, triphenylmethyl, tetrahydrofuran-2-yl, tetrahydropyran-2-yl, 4-methoxytetrahydrofuran-4-yl, $C_{2-6}$n-alkanoly. Especially preferred are trisubstituted silyl radicals in particular diphenyl-t-butylsilyl ( = Pro').

Deprotection is carried out in conventional manner e.g. by cleavage under mild conditions such as employing e.g. for removal of a diphenyl-t-butylsilyl a fluoride reagent e.g. tetra-n-butylammonium fluoride in an anhydrous inert organic medium preferably tetrahydrofuran containing glacial acetic acid at temperatures of 20° to 60° C, especially 20° to 25° C. Preferably 1-4 moles of fluoride are used with 1.2 to 1.5 moles of glacial acetic acid to each mole of fluoride.

The required starting materials may be prepared for example as illustrated in the following reaction schemes or in the examples hereinafter.

Further suitable reaction conditions are disclosed e.g. in EP 114027 and 117228 including the examples thereof.

**Abreviations:**

| | |
|---|---|
| AIO - | anhydrous inert organic solvent |
| ES - | ether solvent e.g. diethylether, 1,2-diethoxyethane, 1,2-dimethoxyethane, THF or mixtures thereof |
| HC - | hydrocarbon solvent e.g. benzene, toluene, xylene or mixtures thereof |
| HLA - | halogenated lower alkane solvent e.g. $CCl_4$, $CHCl_3$, 1,1-dichloroethane, 1,2-dichloroethane, methylene chloride, 1,1,2,-trichloroethane |
| IO - | inert organic solvent |
| THF - | tetrahydrofuran |
| DMSO - | dimethylsulfoxide |
| LDA - | lithiumdiisopropylamide |
| nBuLi - | n-butyllithium |
| DMF - | dimethylformamide |
| DIBAH - | diisobutylaluminium hydride |

**Variables not previously defined**

each

| | |
|---|---|
| $R_{15}$ | is independently $C_{1-3}$alkyl preferably n-$C_{1-3}$alkyl, especially $C_{1-2}$alkyl |
| $X_1$ | is $-CH_2-$ or $-(CH_2)_2-$ |
| $X_2$ | is a direct bond or $-CH_2-$ |
| $X_3$ | is -CH = CH-, -CH = CH-$CH_2$ - or -$CH_2$-CH = CH- preferably (E)-CH = CH-, (E)-CH = CH-$CH_2$- or -$CH_2$-CH = CH- especially (E)-CH = CH-, |
| $X_4$ | is $-(CH_2)_2-$ or $-(CH_2)_3-$ especially $-(CH_2)_2-$ |

| | |
|---|---|
| $X_5$ | is $(CH_2)_m$- or (E)-CH=CH- (m-0,1,2 or 3) |
| Y | is Cl, Br or I |
| Y' | is Cl or Br |

A is 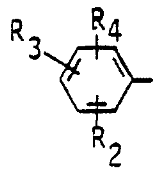

B is 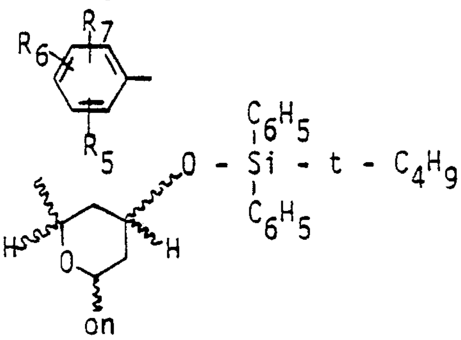

Lac -

on = $OCH_3$ ($Lac_1$)
OH ($Lac_2$)
O = ($Lac_3$)

Pro' = 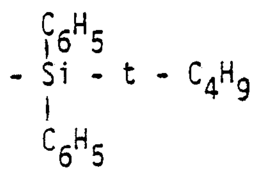

$R_{13}'$ is $C_{1-3}$alkyl, n-butyl, i-butyl, t-butyl or benzyl

REACTION SCHEME I

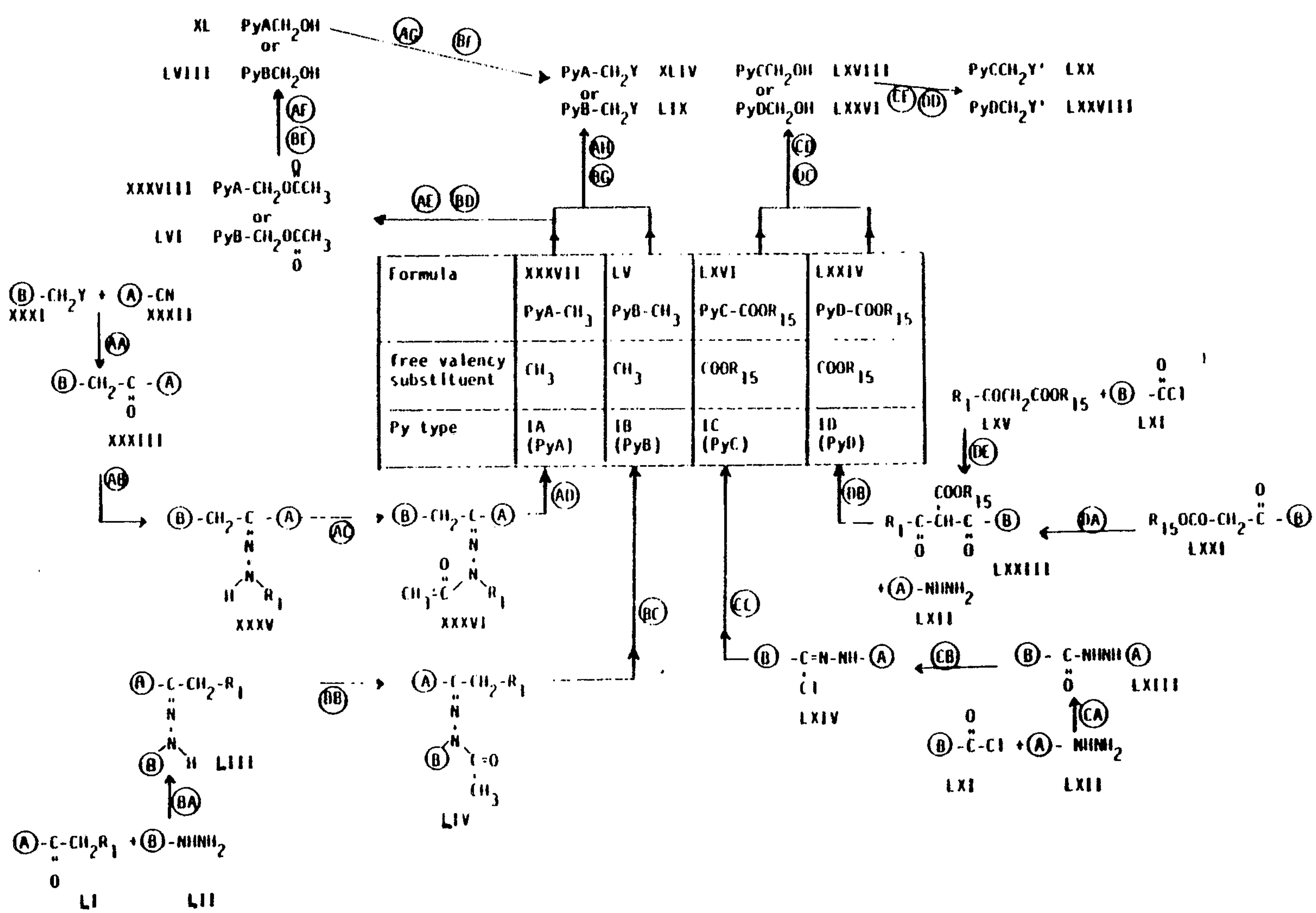

| Formula | XXXVII<br>PyA-CH3 | LV<br>PyB-CH3 | LXVI<br>PyC-COOR15 | LXXIV<br>PyD-COOR15 |
|---|---|---|---|---|
| free valency substituent | CH3 | CH3 | COOR15 | COOR15 |
| Py type | IA (PyA) | IB (PyB) | IC (PyC) | ID (PyD) |

REACTION SCHEME II

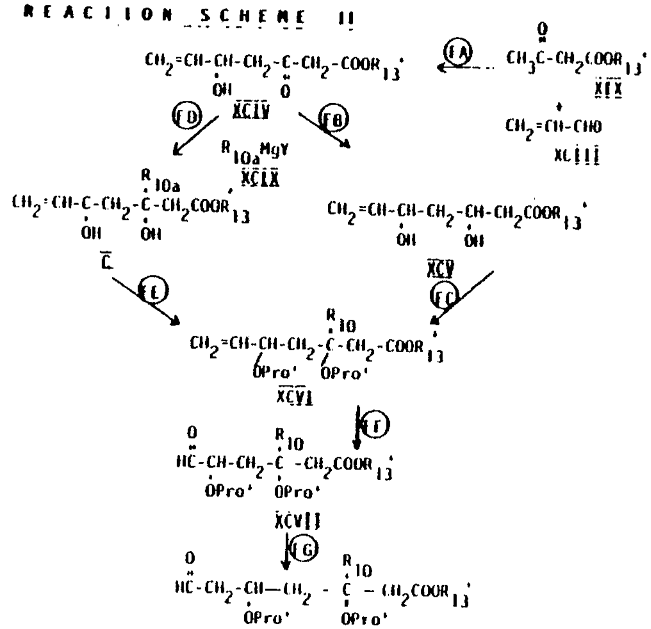

XCVIII (= IX when $X_2$ = $(CH_2)$)

REACTION SCHEME IV

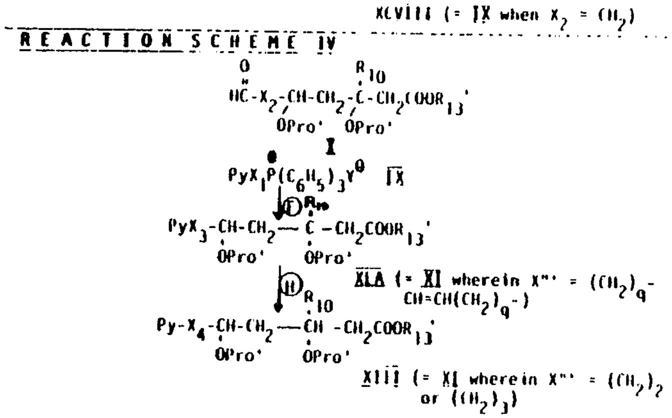

REACTION SCHEME III

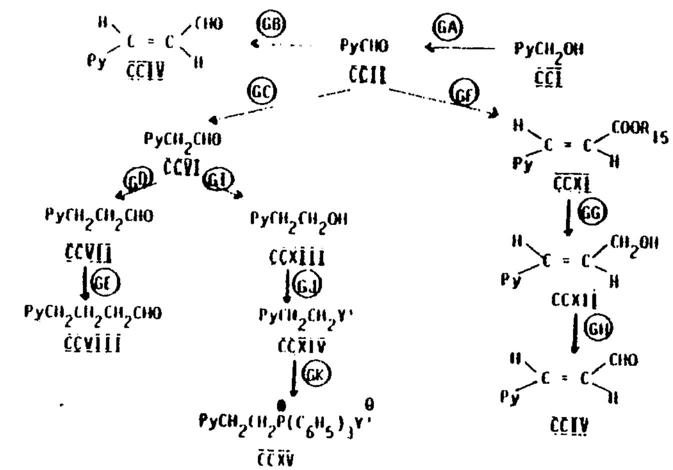

The aldehydes produced according to this reaction scheme and analogues thereof may be employed for preparing the starting materials of formulae XX and XVIII (cf. e.g. EP 117228)

REACTION SCHEME V

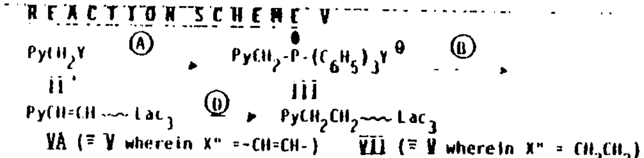

REACTION SCHEME VI

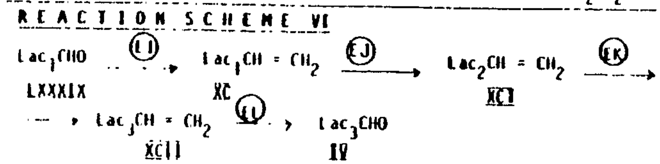

**EXAMPLES OF REACTION CONDITIONS**

| REACTION/TYPE | COMMENTS | TEMPERATURE | SOLVENT | INERT ATMOSPHERE |
|---|---|---|---|---|
| A<br>and analogously for GK | using $(C_6H_5)_3P$ | 60° to reflux pref. ≤150°, esp. 75-90° | AIO pref. HC | Yes |
| B (Wittig)<br>and analogously for F | 1. Strong base e.g. NaH, nBuLi<br>2. Add $Lac_3CHO$ IV (particularly suited for 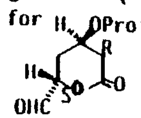 IVa) | 1. -40° to 5° pref. -35° to -20°<br>2. -55° to 25° pref. -35° to -5° | AIO pref. HC e.g. benzene, toluene or pref. ES e.g. THF<br>" " " | Yes<br>" |
| D<br>(Hydrogenation)<br>and analogously for H | $H_2$ at raised pressure (e.g. 30-60 psi)<br>$PtO_2$ as catalyst<br>(Product of B prepared using IVa yields 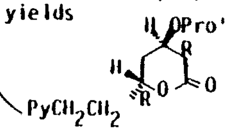) | 20 to 25° | Lower alkanol e.g. $C_2H_5OH$ | - |
| AA<br>(Grignard+Hydrolysis) | 1. XXXI + Mg<br>2. Add XXXII<br>3. Excess aq. inorganic acid e.g. 10% HCl | 1. 10° to reflux pref. 30-38°<br>2. 10° to reflux, 10° to 75°, pref. 20-40°, esp. 20-25°.<br>3. 0-25° | 1,2. Anh. ES esp. $(C_2H_5)_2O$ or THF<br>3. Inert aq. org. e.g. as 1. 2. + $H_2O$ | Yes |

| REACTION/TYPE | COMMENTS | TEMPERATURE | SOLVENT | INERT ATMOSPHERE |
|---|---|---|---|---|
| AB | $R_1$-$NHNH_2$ XXXIV + $CH_3COOH$ as catalyst | 60° to 100° pref. 70-90° | 10 optionally + ≤ ca. 10% $H_2O$ e.g. lower alkanol such as $CH_3OH$, $C_2H_5OH$ e.g. 95% $C_2H_5OH$ | - |
| AC (Acylation) | $CH_3COCl$ + base e.g. tertiary amine such as triethylamine | 0°-5° rising to 20-25° | as B | - |
| AD (Cyclisation) | $OH^{\ominus}$ e.g. hydroxide such as NaOH or KOH | 50-90° pref. 70-80° | A10 e.g. higher boiling ES pref. bis(2-methoxyethyl)- or bis(2-ethoxyethyl)-ether | Yes |
| AE | $Pb(OAc)_4$ | 20-80° | Glacial $CH_3COOH$ or benzene | Yes |
| AF (Hydrolysis) | as AD | 20-50° | Inert aq. org. e.g. $H_2O$ + lower alkanol pref. $H_2O$ + $CH_3OH$ or esp. $C_2H_5OH$ | - |
| AG (Halogenation) and analogously for CE and GJ | $SOY_2'$ or $PY_3'$ (Y in product = Y') | -10°-80° | A10 pref. ES e.g. diethylether or THF or HLA e.g. methylene chloride or HC e.g. benzene | - |

| REACTION/TYPE | COMMENTS | TEMPERATURE | SOLVENT | INERT ATMOSPHERE |
|---|---|---|---|---|
| AH (Halogenation) | N-Br- or N-Cl- or N-I-succinimide + organic peroxide e.g. di-benzoyl peroxide as catalyst | 50-100° pref. 70-90° | AIO pref. HLA esp. $CCl_4$ | Yes |
| BA | $CH_3COOH$ as catalyst | as AB | as AB | - |
| BB-BG | as AC - AH | as AC - AH | as AC - AH | as AC - AH |
| CA (Acylation) | tertiary amine e.g. triethylamine | -10° to 0° rising to 20-25° | AIO e.g. ES pref. diethyl-ether or THF | - |
| CB | $PCl_5$ | 30-40° pref. 35° | AIO e.g. ES pref. diethyl-ether | Yes |
| CC | Base e.g. $NaOC_2H_5$ + $R_1$-CO-$CH_2COOR_{15}$ (LXV) | 20-25° | Anh. lower alkanol e.g. abs. $C_2H_5OH$ | Yes |
| CD (Reduction) | Reducing agent e.g. $LiAlH_4$ | -5° to 25° pref. -5° to 5° rising to 20-25° | as CB | Yes |
| DA | 1. Strong base e.g. NaH<br>2. $R_1$-CO-Cl (LXXII) | 1. -5° to 5°<br>2. -5° to 5° rising to 20-25° | AIO pref. ES, esp. THF<br>" " " " " | Yes<br>" |

| REACTION/TYPE | COMMENTS | TEMPERATURE | SOLVENT | INERT ATMOSPHERE |
|---|---|---|---|---|
| DB (Cyclisation) | - | 20° to 40° pref. 20° to 25° | Glacial $CH_3COOH$ or DMSO | Yes |
| DC and DD | as CD and AG | as CD and AG | as CD and AG | as CD and AG |
| DE | as DA | as DA | as DA | as DA |
| EI (Wittig) | 1. $(C_6H_5)_3P=CH_2$ (prepared from methyltriphenylphosphonium bromide or pref. iodide + n-BuLi<br>2. Add LXXXIX | -30° to 25° pref. -5° to 5°<br>-10° to 10° pref. -5° to 5° rising to 20° to 25° | Anh. THF<br>Anh. THF | Yes<br>Yes |
| EJ (Hydrolysis) | - | 60° to 70° | Glacial $CH_3COOH$ + THF + $H_2O$ pref. 3 : 2 : 2 in a quantity to achieve a large molar excess e.g. 40-60 mole $CH_3COOH$ | - |
| EK (Oxidation) | Mild oxidising agent e.g. pyridinium chlorochromate | 20° to 25° | Anh. methylene chloride | - |
| EL (Ozonolysis) | $O_3$ in excess quench with $(CH_3)_2S$ or $(C_6H_5)_3P$ | -80° to -70° pref. -78° | $C_{1-3}$alkanol esp. $CH_3OH$ or HLA esp. methylene chloride or ethylacetate | - |

| REACTION/TYPE | COMMENTS | TEMPERATURE | SOLVENT | INERT ATMOSPHERE |
|---|---|---|---|---|
| FA | 1. Strong base e.g. LDA or NaH followed by n-BuLi to generate dianion or XIX<br>2. Add XCIII | 1. -50° to 10° pref. -10° to 10°<br>2. -80° to 0° pref. -40° to -20° esp. -35° to -30° rising to 20-25° | as DA<br>as DA | Yes<br>Yes |
| FB (Reduction) | Analogous to process a) ⟶ | ⟶ | ⟶ | ⟶ |
| FC (Silylation) | 2-8 moles pref. 4 moles of Pro'Cl per mole XCV + 2 moles imidazole per mole Pro'Cl | 20° to 30° pref. 20° to 25° | Anh. DMF | Yes |
| FD (Gringard) | $R_{10a}MgY$ XCIX quench on completion e.g. with aq. $NH_4Cl$ | -70° to 25° pref. -50° to 0° | as DA | Yes |
| FE | as FC | as FC | as FC | as FC |
| FF | as EL | as EL | as EL | as EL |

| REACTION/TYPE | COMMENTS | TEMPERATURE | SOLVENT | INERT ATMOSPHERE |
|---|---|---|---|---|
| FG (Wittig) | 1. $(C_6H_5)_3\overset{\oplus}{P}-CH_2OCH_3Cl^{\ominus}$ CCV + strong base e.g. NaH phenyllithium or n-BuLi<br>2. Add XCVII<br>3. Hydrolysis : excess of strong acid e.g. 70% perchloric | 1. -40° to 0° pref. -35° to-20°<br>2. -30° to 0° pref. -20° to 0°<br>3. 0° to 30° | 1 2. as DA<br>3. aq. acid + ES e.g. perchloric acid + THF | Yes<br>Yes<br>- |
| GA (Oxidation) | pyridinium chlorochromate or pyridinium dichromate or chromium trioxide or activated manganese dioxide pref. in mole excess | 20° to 30° pref. 20° to 25° | HLA e.g. methylene chloride for all except for manganese dioxide where ES e.g. diethylether may also be used | Yes |
| GB | 1. add 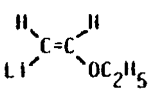 CCIII prepared e.g. from cis-1-ethoxy-2-tri-n-butylstannylethylene and n-BuLi<br>2. Hydrolysis with water and e.g. p-toluenesulfonic acid as catalyst | -80° to -40° pref. -80° to -60°<br>20° to 40°C pref. 20° to 25° | as DA<br>ES + $H_2O$ esp. THF + $H_2O$ | Yes<br>- |

| REACTION/TYPE | COMMENTS | TEMPERATURE | SOLVENT | INERT ATMOSPHERE |
|---|---|---|---|---|
| GE - GE | as FG | as FG | as FG | as FG |
| GF (Wittig) | alt. a)<br>Add $(C_6H_5)_3P{=}CH\text{-}COOR_{15}$ CCIX | 80° to reflux esp. toluene reflux | AIO pref. HC esp. toluene | Yes |
| | alt. b) 1. $(R_{15}O)_2PO\text{-}CH_2\text{-}COOR_{15}$ CCX<br>+ strong base e.g. NaH | -20° to 25° pref. -20° to 0° | as DA | Yes |
| | 2. CCII + product of 1. | -20° to 25° | as DA | Yes |
| GG (Reduction) | 1. $LiAlH_4$, DiBAH<br>2. quench e.g. with aq. $NH_4Cl$ or aq. $Na_2SO_4$ | 0° to 25° pref. 0-10° | as DA | Yes |
| GI (Reduction) | a) as "a)"<br>b) as CD | a) as "a)"<br>b) as CD | a) as "a)"<br>b) as CD | -<br>Yes |

The conditions given hereinabove are largely conventional for such reactions and can be varied in conventional manner according to the particular intermediates/end products. This applies e.g. to molar ratios, temperature, reaction times and the like which are chosen according to principles well established in the art on the basis of reactants and conditions employed.

Intermediates, the production of which is not described above, are either known or may be prepared according to or analogously to known methods e.g. as described in EP-A-114027 and 117228 including the examples thereof.

Reaction products, both intermediates and final, can be isolated (e.g. from compound mixtures or reaction mixtures) and purified in conventional manner whereby intermediates can, where appropriate, be employed directly in a subsequent reaction.

Mixtures of stereoisomers (cis , trans and optical) can be separated by conventional means at whatever

stage of synthesis is appropriate. Such methods include re-crystallisation, chromatography, formation of esters with optically pure acids and alcohols or of amides and salts with subsequent reconversion under retention of optical purity. For example diastereoisomeric (-)-α-naphthylphenylmethylsilyl derivatives of a lactone type end product of formula I may be separated by conventional means.

Salts may be prepared in conventional manner from free acids, lactones and esters and vice-versa. Whilst all salts are covered by the invention pharmaceutically acceptable salts especailly sodium, potassium and ammonium particularly sodium salts are preferred.

The various forms of the compounds of formula I are by virtue of their interconvertability useful as intermediates in addition to the use set out below.

Also within the scope of this invention are the intermediates of formulae V, VII, XI, XIII, XVIIE, XX, and CCIV, .

The preferences for each variable are the same as set out for formula I and preferred groups of compounds correspond to those listed for formula I as appropriate to and consistent therewith.

The compounds of formula I possess pharmacological activity in particular as competitive inhibitors of 3-hydroxy-3-methyl-glutaryl coenzyme A (HMG-CoA) reductase and as a consequence are inhibitors of cholesterol biosynthesis as demonstrated in the following tests.

**Test A**: In Vitro Microsomal Assay of HMG-CoA Reductase Inhibition: As described in EP 114027.

**Test B**: In Vivo Cholesterol Biosynthesis Inhibition As described in EP 114027.

The compounds are thus indicatd for use as hypolipoproteinemic and anti-atherosclerotic agents.

An indicated suitable daily dosage for use in the treatment of hyperlipoproteinemia and atherosclerosis is from 0.1 to 2000 mg preferably 0.1 to 150 mg suitably administered in divided dosages one to four times daily or in retard form. A typical dosage unit for oral administration may contain 0.025 to 500 mg.

The compounds of formula I may be administered in similar manner as known compounds suggested for use in such indications e.g. Compactin or Mevinolin.The suitable daily dosage for a particular compound will depend on a number of factors such as its relative potency of activity. It has, for example been determined that the preferred compounds (compounds of examples nos. 3, 14) obtained an $ED_{50}$ of 0.05, 0.013 mg/kg respectively in Test B compared with 3.5 mg/kg for Compactin and 0.41 mg/kg for Mevinolin. It is therefore indicated that the compounds may be administered at similar or significantly lower dosages than conventionally proposed for Compactin or Mevinolin.

They may be administered in free acid form or in the form of a physiologically-acceptable ester e.g. one which is also physiologically hydrolysable or a lactone thereof or in pharmaceutically acceptable salt form.

The invention therefore also concerns a method of treating hyperlipoproteinemia or atherosclerosis by administration of a compound of formula I in free acid form or in the form of a physiologically-hydrolysable and -acceptable ester or a lactone thereof or in pharmaceutically acceptable salt form as well as such compounds for use as pharmaceuticals e.g. as hypolipoproteinemic and anti-atherosclerotic agents.

The compounds may be administered alone, or in admixture with a pharmaceutically acceptable diluent or carrier, and, optionally other excipients, and administered orally in such forms as tablets, elixirs, capsules or suspensions or parenterally in such forms as injectable solutions or suspensions.

The preferred pharmaceutical compositions from the standpoint of ease of preparation and administration are solid compositions, particularly tablets and hard-filled or liquid-filled capsules.

Such compositions also form part of the invention.

The following examples, in which all temperatures are in °C illustrate the invention.

**EXAMPLE 1**

(E)-Trans -6S-(2'-[4"-(4'"-fluorophenyl)-1"-(1'"-methylethyl)-3"-phenyl-1H-pyrazol-5"-yl]ethenyl)-4R-hydroxy-3,4,5,6-tetrahydro-2H-pyran-2-one (compound no. 1 of formula IAb, trans isomer: 4R,6S form) ($R_1$ = i-$C_3H_7$, $R_5$ = p-F, $R_2$-$R_4$,$R_6$,$R_7$ = H, $R_{10}$ = H, X = (E)-CH=CH).

**Step 1**(Reaction AA)

α-(4-Fluorophenyl)acetophenone (Compound XXXIIIa) (type XXXIII; $R_5$ = p-F, $R_2$-$R_4$,$R_6$,$R_7$ = H).

5.27 ml (42 mmoles) of 4-fluorobenzyl bromide is slowly added to 928mg (38 mmoles) of magnesium turnings stirred in 38 ml of anhydrous diethyl ether under nitrogen over a period of 45 minutes at a rate such that the reaction mixture gently refluxes. The reaction mixture is allowed to cool to 20°-25°, 2.96 ml (29 mmoles) of benzonitrile in 5 ml of anhydrous diethyl ether is added over a period of 5 minutes, and the

reaction mixture is stirred at 20°-25° for 3 hours, the reaction mixture being maintained under nitrogen throughout. The reaction mixture is slowly poured into ice cold 10% hydrochloric acid, and the organic phase is separated. The aqueous phase is extracted five times with diethyl ether and twice with ethyl acetate, and the organic phases are combined, washed once with saturated sodium bicarbonate solution, washed once with saturated sodium chloride solution, dried over anhydrous magnesium sulfate and evaporated at reduced pressure. The residue is dissolved in 450 ml of hot petroleum ether, and the solution is filtered and cooled to obtain the product as an off-white solid, m.p. 109°-110°.

Step 2(Reaction AB)

1-[2'-(4"-Fluorophenyl)-1'-phenylethylidene]-2-(1'-methylethyl)-hydrazine (Compound XXXVa) (type XXXV: $R_5$ = p-F, $R_2$-$R_4$,$R_6$,$R_7$ = H, $R_1$ = i-$C_3H_7$).

A mixture of 3 g (14.0 mmoles) of Compound XXXIIIa, 2.45 ml (28 mmoles) of isopropylhydrazine and 0.5 ml of acetic acid in 23 ml of 95% ethanol is stirred at 80° for 1.4 hours. The reaction mixture is cooled, most of the solvent is evaporated at reduced pressure and methylene chloride is added. The methylene chloride solution is washed with saturated sodium chloride solution, dried over anhydrous sodium sulphate and evaporated to dryness at reduced pressure. Toluene is added to the residue, and the mixture is evaporated to dryness at reduced pressure to obtain the product as a somewhat cloudy yellow liquid.

Isopropylhydrazine is synthesized as follows: 9.39 ml (0.1 mole) of 2-bromopropane is slowly added over a period of 2 hours to 48.4 ml (1 mole) of hydrazine hydrate stirred under nitrogen at 20°-25°. During the addition the temperature rises to as high as 40°. The reaction mixture is stirred under nitrogen at 60° for 3.25 hours, cooled to 20°-25° and continuously extracted with diethyl ether for 20 hours. The diethyl ether is evaporated at reduced pressure to obtain the crude product which is used as it is. B.p. 106°-107°. (742 mm.Hg.)

Step 3 (Reaction AC)

1-Acetyl-2-[2'-(4"-fluorophenyl)-1'-phenylethylidene]-1-(1'-methylethyl)-hydrazine (Compound XXXVIa) (type XXXVI; $R_5$ = p-F, $R_2$-$R_4$,$R_6$,$R_7$ = H, $R_1$ = i-$C_3H_7$).

3.9 ml (28 mmoles) of triethylamine is added to a solution of 3.97g (≦ 14 mmoles) of crude Compound XXXVa in 140 ml of toluene, the mixture is cooled to 0°-5°, 1.24ml (17.5 mmoles) of acetyl chloride is added, and the reaction mixture is stirred for 1.5 hours with gradual warming to 20°-25°. 500 ml of diethyl ether is added, and the solution is filtered through anhydrous sodium sulfate and evaporated to dryness at reduced pressure. Toluene is added, and the mixture is evaporated to dryness at reduced pressure. The residue is chromatographed on 160 g of silica gel utilizing 75% diethyl ether/n-hexane as the eluant. The fractions containing the product (as determined by thin layer chromatography) are combined and evaporated at reduced pressure to obtain the product as a yellow oil.

Step 4(Reaction AD)

4-(4'-Fluorophenyl)-5-methyl-1-(1'-methylethyl)-3-phenyl-1H-pyrazole (Compound XXXVIIa) (type XXXVII; $R_5$ p-F; $R_2$-$R_4$,$R_6$,$R_7$ = H, $R_1$ = i-$C_3H_7$).

A mixture of 1.6 g (5.1 mmoles) of Compound XXXVIa and 0.656 g (10.2 mmoles) of potassium hydroxide in 51 ml of bis -(2-methoxyethyl) ether is stirred at 80° under nitrogen for 2 hours, an additional 0.75 g of potassium hydroxide is added, and the reaction mixture is stirred under nitrogen at 80° for 2 hours and at 20°-25° for 16 hours and poured into 300 ml of distilled water. The mixture is extracted three times with 150 ml portions of diethyl ether and once with 200 ml of ethyl acetate. The organic extracts are combined, washed with 500 ml of ice cold 3% hydrochloric acid and twice with saturated sodium chloride solution, dried over anhydrous magnesium sulfate, filtered, concentrated at reduced pressure, and heated at 90°-100° for a few minutes. A small amount of chloroform and n-hexane is added, and the solution is cooled in a sealed container at -78° to obtain the crystalline product, m.p. 139°-140°.

Step 5(Reaction AH)

5-Bromomethyl-4-(4'-fluorophenyl)-1-(1'-methylethyl)-3-phenyl-1H-pyrazole (Compound XLIVa) (type XLIV; $R_5$ = p-F; $R_2$-$R_4$, $R_6$, $R_7$ = H, $R_1$ = i-$C_3H_7$, Y = Br).

A mixture of 0.9 g (3.06 mmoles) of compound XXXVIIa, 654 mg (3.67 mmoles) of N-bromosuccinimide and 37 mg of 5 molar % dibenzoyl peroxide in 61.2 ml of carbon tetrachloride is stirred at 80° under nitrogen

for 45 minutes. The reaction mixture is cooled to 20°-25°, filtered and evaporated to dryness at reduced pressure to obtain the crude product as a pale yellow oil. Petroleum ether is added to partially crystallize the product. An analytical sample is recrystallized from petroleum ether, m.p. 93°-95°.

**Step 6**(Reaction A)

4-(4'-Fluorophenyl)-1-(1'-methylethyl)-3-phenyl-5-triphenylphosphoniummethyl-1H-pyrazole bromide (Compound IIIa) (type III; Py = PyA, $R_1$ = i-$C_3H_7$, $R_5$ = p-F, $R_2$-$R_4$,$R_6$,$R_7$ = H, Y = Br).

A mixture of 0.6 g (1.77 mmoles) of Compound XLIVa (the oily product of the preceding step) and 0.58 g (2.21 mmoles) of triphenylphosphine in 42 ml of toluene is stirred at 80° under nitrogen for 45 minutes. The reaction mixture is allowed to cool to 20°-25°, and the solid white product is collected by filtration and washed with diethyl ether.

**Step 7**(Reaction EL and B)

(E)-Trans -4R-(1',1'-dimethylethyl-diphenylsilyloxy)-6S-(2'-[4"-(4"'-fluorophenyl)-1"-(1'"-methylethyl)-3"-phenyl-1H-pyrazol-5"-yl]ethenyl)-3,4,5,6-tetrahydro-2H-pyran-2-one (compound Va) and its (Z) isomer (compound Vb) (type V; Py = PyA, $R_1$ = i-$C_3H_7$, $R_5$ = p-F, $R_2$-$R_4$,$R_5$,$R_6$ = H, Pro = Pro', X" = (E)-CH=CH or (Z)-CH=CH trans isomer; 4R,6S form).

(a) Trans -4R-(1',1'dimethylethyl-diphenylsilyloxy)-6S-formyl-2H-pyran-2-one (Compound IVa: type IV trans isomer; 4R,6S form): Ozone is bubbled through a solution of 200 mg (0.526 mmole) of trans -4R-(1',1'-dimethylethyl-diphenylsilyloxy-6S-vinyl-3,4,5,6-tetrahydro-2H-pyran-2-one (Compound XCIIa; type XCII trans isomer; 4R,6S form) in 26 ml of methylene chloride stirred at -78° until the solution has a bluish tint (about 5 minutes), and 0.5 ml of dimethyl sulfide is added by syringe to quench the rection mixture. The mixture is warmed to 20°-25°, the solvent is evaporatd at reduced pressure, and any remaining solvent is evaporated under high vacuum to obtain the product as an oil.

(b) 501 mg (0.789 mmol) of Compound IIIa is dried under high vacuum for 2 hours (to remove any trace of solvent present) and dissolved in 7.89 ml of dry tetrahydrofuran. The solution is stirred at-30° under nitrogen and 570 µl of 1.7M.n-butyllithium/n-hexane (0.969 mmole) is added by syringe to obtain a solution of the ylid (compound offormula IIIa having a $-CH=P(C_6H_5)_3$ group in lieu of the $-CH_2P^{\oplus}(C_6H_5)_3Br^{\ominus}$ group).

(c) The ylid solution of Part (b) is added by syringe to a solution of Compound IVa (from Part (a)) in 5.26 ml of dry tetrahydrofuran stirred at -30° under nitrogen, and the reaction mixture is stirred under the same conditions for 1 hour and quenched with 5 ml of saturated ammonium chloride solution. The mixture is extracted with 150 ml of diethyl ether, and the diethyl ether extract is washed twice with saturated sodium chloride solution, dried over anhydrous magnesium sulfate, filtered and evaporated to dryness at reduced pressure to obtain the crude product as a brown foam.

(d) The brown foam from Part (c) is triturated three times with 50% diethyl ether/n-hexane, and the diethyl ether/n-hexane solutions are combined and evaporated at reduced pressure to an oil. The oil is chromatographed on 40 g of silica gel utilizing 50% diethyl ether/n-hexane as the eluant, and the fractions containing each product are combined and evaporated at reduced pressure to obtain the (E) olefin (Compound Va) and the (Z) olefin (Compound Vb) as oils. The (E) olefin is eluted prior to the (Z) olefin.

**Step 8**(Reaction c))

(E)-Trans -6S(2'-[4"-(4"'-fluorophenyl)-1"-(1"-(1"'-methylethyl)-3"-phenyl-1H-pyrazol-5"-yl]ethenyl)-4R-hydroxy-3,4,5,6-tetrahydro-2H-pyran-2-one (Compound no. 1).

16.2 µl (0.28 mmole) of glacial acetic acid is added dropwise by syringe to a solution of 36.6 mg (0.056 mmole) of Compound Va in 2.8 ml of tetrahydrofuran stirred at 20°-25° under nitrogen. 224 µl of 1M. tetra-n-butylammonium fluoride/tetrahydrofuran (0.224 mmole) is added by syringe, and the reaction mixture is stirred at 20-25° under nitrogen for 3.75 hours and poured into 20 ml of ice cold water. The mixture is extracted four times with diethyl ether, and the diethyl ether extracts are combined, washed with saturated sodium bicarbonate solution, washed with saturated sodium chloride solution, dried over anhydrous magnesium sulfate, filtered and evaporated to dryness at reduced pressure. The residue is washed with 50% diethyl ether/petroleum ether to obtain the product as a white solid, m.p. 216° (yellows and shrinks at 214°). $[\alpha]_D^{25}$ = -6.86° ($CH_3COCH_3$, c = 0.35).

EXAMPLE 2

(Z)-Trans-6S-(2'-[4"-(4'''-fluorophenyl)-1"-(1'''-methylethyl)-3"-phenyl-1H-pyrazol-5"-yl]ethenyl)-4R-hydroxy-3,4,5,6-tetrahydro-2H-pyran-2-one (Compound no. 2 of formula IAb) (substituents as compound no. 1 except X = (Z)-CH=CH).
The product is obtained from 34 mg (0.052 mmole) of Compound Vb by the process of Step 8 of Example 1 utilizing 15.0 µl. (0.26 mmole) of glacial acetic acid, 208 µl of 1M. tetra-n-butyl-ammonium fluoride/tetrahydrofuran (0.208 mmole) and, as the solvent, 26 ml of tetrahydrofuran except that the reaction time is 24-28 hours, m.p. 190° $[\alpha]_D^{25}$ = +171.37° ($CH_2Cl_2$, c = 0.51).

EXAMPLE 3

Sodium erythro -(E)-3R,5S-dihydroxy-7-[4'(4"-fluorophenyl)-1'-(1"-methylethyl)-3'-phenyl-1H-pyrazol-5'-yl]-hept-6-enoate (compound no.3 of formula IAa in sodium salt form; erythro isomer; 3R,5S form) (substituents as compound no. 1). (Reaction e)).
50.16 ul of 0.5 N. sodium hydroxide solution (0.0251 mmole) is added by syringe to a solution of 11.1 mg (0.0264 mmole] of Compound no.1 in 2 ml of methanol stirred at 20-25° and the reaaction mixture is stirred under nitrogen at this temperature for 3 hours. The methanol is evaporated at reduced pressure, and the residue is dissolved in 2 ml of water. The aqueous solution is carefully extracted with diethyl ether, and the last traces of diethyl ether are removed at reduced pressure. The aqueous solution is cooled to -78° and freeze-dried under high vacuum to obtain the product as a free-flowing pale yellow foam begins to darken at 166°; almost completely a black char at 215°.

N.M.R. ($CDCl_3$ $CD_3OD$): 1.53 (d (J=6.5 Hz.), 6H), 1.5 (m,2H), 2.3 (m,2H), 4.1 (bm,1H), 4.3 (bm,1H), 4.65 (m (J=6.5 Hz.), 1H), 5.62 (dd ($J_1$=16 Hz., $J_2$=5 Hz.), 1H), 6.48 (d (J=16 Hz.), 1H), 6.9-7.4 (m,9H).

EXAMPLE 4

Sodium erythro-(Z)-3R,5S-dihydroxy-7-[4'-(4"fluorophenyl)-1'-(1" -methylethyl)-3'-phenyl-1H-pyrazol-5'-yl]-hept-6-enoate (Compound no.4 of formula IAa in sodium salt form; erythro isomer; 3R,5S form) (substituents as compound no. 2) (Reaction e)).
Analogous to Example 3 starting from Compound no. 2.

EXAMPLE 5 and 6

Methyl erythro -(E)-3,5-dihydroxy-7-[4'-(4"-fluorophenyl)-1'-(1"-methylethyl)-3'-phenyl-1H-pyrazol-5'-yl]hept-6-enoate (Compound no. 5 of formula IAa in methyl ester form; erythro isomer).
(E)-Trans -6-(2'[4"-(4'''-fluorophenyl)-1"-(1'''-methylethyl)-3"-phenyl-1H-pyrazol-5"-yl]ethenyl)-4-hydroxy-3,4,5,6-tetrahydro-2H-pyran-one (compound no. 6 of formula IAb; trans isomer) ($R_1$ = $iC_3H_7$, $R_5$ = p-F, $R_2$-$R_4$,$R_6$,$R_7$=H, $R_{10}$ = H, X = (E)-CH = CH).

Step 1(Reaction F)

Methyl erythro -(E)-3,5-[di-(1',1'-dimethylethyl-diphenylsilyloxy)]-7-[4'-(4"-fluorophenyl)-1'-(1"-methylethyl)-3'-phenyl-1H-pyrazol-5'-yl]-hept-6-enoate (Compound XIa; erythro isomer) (Py = PyA, X''' = (E)-CH = CH, $R_5$ = p-F, $R_2$-$R_4$,$R_6$,$R_7$ = H, $R_{10}$ = H, $R_1$ = i-$C_3H_7$, Pro = Pro',$R_{13}$=$CH_3$). The product is obtained from 0.7 g (1.1 mmoles) of Compound IIIa, 0.7g (1.1 mmoles) of methyl erythro -3,5-di-(1',1'-dimethylethyl-diphenylsilyloxy)-6-oxohexanoate, 1.4 ml of 1.6M. n-butyllithium/n-hexane (2.2 mmoles) and 25 ml of dry tetrahydrofuran as the solvent substantially in accordance with Parts (b) and (c) of Step 7 of Example 1.

Step 2(Reaction d))

Methyl erythro -(E)-3,5-dihydroxy-7-[4'-(4"-fluorophenyl)-1'-(1"-methylethyl)-3'-phenyl-1H-pyrazol-5'-yl]hept-6-enoate (Compound no. 5) and (E)-Trans -6-(2'-[4"-(4'''-fluorophenyl)-1"-(1'''-methylethyl)-3"-phenyl-1H-pyrazol-5"-yl]ethenyl)-4-hydroxy-3,4,5,6-tetrahydro-2H-pyran-2-one (Compound no. 6).

(a) 3.6 ml of 1M. tetra-n-butylammonium fluoride/tetrahydrofuran (3.6 mmoles) is added to a mixture of 0.83 g (0.9 mmole) of crude Compound XIa, 0.42 ml (7.2 mmoles) of glacial acetic acid and 20 ml of tetrahydrofuran, and the reaction mixture is stirred at 20°-25° for 24 hours and poured into saturated

sodium bicarbonate solution. The mixture is extracted with diethyl ether, the diethyl ether extract is dried over anhydrous magnesium sulfate, and the diethyl ether is evaporated at reduced pressure. The residue is chromatographed on a silica gel column (2.5 cm x 20.3 cm) utilizing 7:2:1 methyl t-butyl ether/n-hexane/acetone as the eluant. The fractions containing the first component of the major product (as determined by thin layer chromatography) are combined and evaporated at reduced pressure to obtain Compound no. 5 as an oil.

N.M.R. ($CDCl_3$): 1.46-1.58 (m, 2H), 1.58 (d, 6H), 2.46 (d, 2H), 3.10-3.70 (bm, 2H), 3.72 (s, 3H), 4.20 (m, 1H), 4.42 (m, 1H), 4.65 (m, 1H), 5.60 (dd, 1H), 6.52 (d, 1H), 7.00 (t, 2H), 7.12-7.25 (m, 5H), 7.35-7.40 (m, 2H).

The product is a racemate that may be resolved to obtain the 3R,5S and 3S,5R enantiomers.

(b) The fractions containing the second component of the major product (see Part (a)) are combined and evaporated at reduced pressure to obtain Compound no. 6 as crystalline needles. The product is a racemate that may be resolved to obtain the 4R,6S and 4S,6R enantiomers.

**EXAMPLE 7**

Sodium erythr o-(E)-3,5-dihydroxy-7-[4'-(4"-fluorophenyl)-1'-(1"-methylethyl)-3'-phenyl-1H-pyrazol-5'-yl]-hept-6-enoate (Reaction e)) (Compound no. 7; as compound no. 5 but in sodium salt rather than methyl ester form).

A mixture of 11 mg (0.026 mmole) of Compound no. 6, 0.05 ml of 0.5N. sodium hydroxide (0.25 mmole) and 5 ml of 95% ethanol is stirred at 20°-25° for 2 hours and evaporated to dryness at reduced pressure. The residue is dissolved in about 3 ml of water, and the solution is washed twice with diethyl ether and freeze dried to obtain the product as a solid foam.

N.M.R. ($CDCl_3$ + $CD_3OD$): 1.4-1.5 (m, 2H), 1. 58 (d, 6H), 2.18-2.5 (m,2H), 4.1 (m, 1H), 4.36 (m, 1H), 4.68 (m, 1H), 5.65 (dd, 1H), 6.51 (d, 1H), 6.92-7.05 (m, 2H), 7.10-7.40 (m, 7H).

The product is a racemate that may be resolved to obtain the 3R,5S and 3S,5R enantiomers.

**EXAMPLE 8**

Ethyl (E)-3,5-dihydroxy-7-[3'-(4"-fluorophenyl)-5'-(1"-methylethyl)-1'-phenyl-1H-pyrazol-4'-yl]hept-6-enoate (Compound no. 8 of formula ICa in ethyl ester form) ($R_1$ = i-$C_3H_7$, $R_5$ = p-F, $R_2$-$R_4$,$R_6$,$R_7$ = H, $R_{10}$ = H, X = (E)-CH=CH).

**Step 1**(Reaction CA)

4-Fluorobenzoic acid-N'-phenylhydrazide (Compound LXIIIa) (type LXIII; $R_5$ = p-F, $R_2$-$R_4$,$R_5$,$R_6$ = H).

30 ml (0.25 mole) of 4-fluorobenzoyl chloride is added dropwise over a 30 minute period to a mixture of 25 ml (0.25 mole) of phenylhydrazine and 35 ml (0.25 mole) of triethylamine in 500 ml of anhydrous diethyl ether stirred at -10° under nitrogen. The reaction mixture is allowed to warm to 20°-25° and stirred under nitrogen for 3 hours. The obtained solids are collected by filtration, washed with diethyl ether and dissolved in about 600 ml of methylene chloride. The methylene chloride solution is filtered and evaporated at reduced pressure to near dryness, and n-hexane is added to obtain the crude solid product. The crude product is dissolved in tetrahydrofuran and filtered to remove the triethylamine hydrochloride, the tetrahydrofuran is evaporated at reduced pressure, and the obtained product is recrystallized from acetone, m.p. 181°-186°.

**Step 2**(Reaction CB)

4-Fluoro-N-phenylbenzenecarbohydrazonoyl chloride (Compound LXIVa) (type LXIV; $R_5$ = p-F, $R_2$-$R_4$,$R_6$,$R_7$ = H).

A mixture of 8.6 g (41 mmoles) of phosphorus pentachloride, 8.0 g (35 mmoles) of Compound LXIIIa and 60 ml of anhydrous diethyl ether is refluxed under nitrogen for 16 hours and cooled to 20°-25°, a solution of 15 g of phenol in 20 ml of diethyl ether is added, the mixture is stirred for 5 minutes, 15 ml of methanol is added very slowly (exothermic reaction), and the mixture is concentrated at 70°-75° and cooled for 16 hours in a refrigerator to obtain the crystalline product which is washed with cold 5% water/acetone, m.p. 118°-121°.

Step 3(Reaction CC)

Ethyl 3-(4'-fluorophenyl)-5-(1'-methylethyl)-1-phenyl-1H-pyrazole-4-carboxy-late (Compound LXVIa) (type LXVI; Py = PyC; $R_1$ = i-$C_3H_7$, $R_5$ = p-F, $R_2$-$R_4$,$R_6$,$R_7$ = H, $R_{15}$ = $C_2H_5$).
0.28 g (12.0 mmoles) of sodium is dissolved in 40 ml of absolute ethanol stirred at 20°-25°, 2.0 ml (12.0 mmoles) of ethyl isobutyrylacetate is added dropwise with stirring at 20°-25°, the mixture is stirred at 20°-25° for 15 minutes, 3.0 g (12.0 mmoles) of Compound LXIVa is added portion-wise as a solid (the temperature increases to 35°), and the reaction mixture is stirred at 20°-25° for 4 hours, the reaction mixture being maintained under nitrogen throughout. The reaction mixture is quenched with 10 ml of 10% hydrochloric acid, and the mixture is concentrated at reduced pressure (which results in dehydration of an intermediate). The residue is extracted with diethyl ether, and the diethyl ether extract is washed twice with saturated sodium chloride solution, dried over anhydrous magnesium sulfate and evaporated at reduced pressure. The residue is flash chromatographed on a silica gel column (2.5 cm x 20.3 cm) utilizing chloroform as the eluant, and the chloroform is evaporated at reduced pressure to obtain the crude product as a red oil.

Step 4(Reaction CD)

3-(4'-Fluorophenyl)-5-(1'methylethyl)-1-phenyl-1H-pyrazole-4-methanol (Compound LXVIIIa) (type LXVIII; Py = PyC, $R_1$ = i-$C_3H_7$, $R_2$-$R_4$,$R_6$,$R_7$ = H, $R_5$ = p-F).
A solution of 3.0 g (8.5 mmoles) of Compound LXVIa in 20 ml of anhydrous diethyl ether is added to a suspension of 0.65 g (17.1 mmoles) of lithium aluminium hydride in 30 ml of anhydrous diethyl ether stirred at 0°-5° under nitrogen, and the reaction mixture is stirred under the same conditions for 3 hours. The reaction mixture is quenched with about 5 ml of ethyl acetate, and 10 ml of 10% hydrochloric acid is added. The organic phase is separated, and the aqueous phase is extracted with diethyl ether. The organic phases are combined, washed twice with saturated sodium chloride solution, dried over anhydrous magnesium sulphate and evaporated at reduced pressure. the solid residue is recrystallized from 5:1 n-hexane/chloroform to obtain the crystalline product, m.p. 144-147°.

Step 5(Reaction CE)

4-Chloromethyl-3-(4'-fluorophenyl)-5-(1'-methylethyl)-1-phenyl-1H-pyrazole (Compound LXXa) (type LXX; Py = PyC, $R_1$ = i-$C_3H_7$, $R_5$ = p-F, $R_2$-$R_4$,$R_6$,$R_7$ = H, Y' = Cl).
A mixture of 2.0 g (6.5 mmoles) of Compound LXVIIIa, 0.6 ml (8.2 mmoles) of thionyl chloride and 20 ml of benzene (reagent grade) is refluxed under nitrogen for 4 hours and stirred at 20°-25° under nitrogen for 16 hours. The excess thionyl chloride and the benzene are evaporated at reduced pressure, additional benzene is added, the mixture is evaporated at reduced pressure twice, and the residue is dried under high vacuum. Petroleum ether is added to crystallize the product, m.p. 124°-128°.

Step 6(Reaction A)

3-(4'-Fluorophenyl)-5-(1'-methylethyl)-1-phenyl-4-triphenylphosphoniummethyl-1H-pyrazole chloride (Compound IIIb) (type III; Py = PyC, $R_1$ = i-$C_3H_7$, $R_5$ = p-F, $R_2$-$R_4$,$R_6$,$R_7$ = H, Y = Cl).
Analogous to Example 1 Step 6.

Step 7(Reaction F)

Ethyl 3,5-di-(1',1'-dimethylethyl-diphenylsilyloxy)-7-[3'-(4"-fluorophenyl)-5'-(1"-methylethyl)-1'-phenyl-1H-pyrazol-4'-yl]hept-6-enoate (Compound XIb).
Analogous to the process of Step 1 of Examples 5 and 6. The starting material 3,5-di-(1',1'-dimethylethyl-diphenylsilyloxy)-6-oxohexanoate is a ca. 4:1 erythro:threo mixture. The product is obtained as a solid foam. The product is a mixture of the (E)-erythro, (E)-threo, (Z)-erythro and (Z)-threo racemates wherein the ratio of the (E) isomers to the (Z) isomers is about 2:1 and the ratio of the erythro isomers to the threo isomers is about 4:1. The four isomers may be separated by conventional means. Each erythro racemate may be resolved to obtain the 3R,5S and 3S,5R enantiomers and each threo racemate may be resolved to obtain the 3R,5R and 3S,5S enantiomers.

Step 8(Reaction d))

Ethyl (E)-3,5-dihydroxy-7-[3'-(4"-fluorophenyl)-5'-(1"-methylethyl)-1'-phenyl-1H-pyrazol-4'-yl]hept-6-enoate (Compound no. 8).

Analogous to Step 2 of Examples 5 and 6 (20-25° for 72 hrs.,40° for 24 hrs.). The product is an about 4:1 mixture of the (E)-erythro and (E)-threo racemates which may be separated by conventional means. The former may be resolved to obtain the 3R,5S and 3S,5R enantiomers, and the latter may be resolved to obtain the 3R,5R and 3S,5S enantiomers.

## EXAMPLES 9 - 11

(E)-Trans -6-(2'-[3"-(4'''-fluorophenyl)-5"-(1'''-methylethyl)-1"-phenyl-1H-pyrazol-4"-yl]ethenyl)-4-hydroxy-3,4,5,6-tetrahydro-2H-pyran-2-one. (Reactions e) and f)) (Compound no. 11 of formula ICb; trans isomer) ($R_1$ = i-$C_3H_7$, $R_5$ = p-F, $R_2$-$R_4$,$R_6$,$R_7$ = H, $R_{10}$ = H, X = (E)-CH=CH).

(a) A mixture of 65 mg (0.14 mmole) of Compound no. 8, 0.32 ml of 0.5N. sodium hydroxide solution (0.16 mmol) and 10 ml of ethanol is stirred at 20°-25° for 2 hours and evaporated to near dryness at reduced pressure. An about 4:1 mixture of the erythro and threo racemates of Compound no. 9 ( = compound no. 8 in Na-salt rather than ethyl ester form) is present.

(b) The product of Part (a) is dissolved in water and acidified with 10% hydrochloric acid, the solution is extracted with diethyl ether, and the diethyl ether extract is dried over anhydrous magnesium sulphate, filtered and evaporated at reduced pressure to an oil, an about 4:1 mixture of the erythro and threo racemates of Compound no. 10 (compound no. 9 in free acid rather than Na salt form).

(c) The product of Part (b) is dissolved in 10 ml of methylene chloride (reagent grade), 100 mg of N-cyclohexyl-N'-[2'-(N"-methylmorpholinium)ethyl]carbodiimide p-toluenesulfonate is added, and the reaction mixture is stirred at 20°-25° for 3 hours and poured into sodium chloride solution. The mixture is extracted with a 1:1 mixture of diethyl ether and tetrahydrofuran, and the extract is dried over anhydrous magnesium sulfate and evaporated at reduced pressure to a yellow oil. The oil is flash chromatographed on a silica gel column (1.25 cm x 20.3 cm) utilizing 7:2:1 methyl t-butyl ether/n-hexane/acetone as the eluant to obtain the compound no. 11.

N.M.R ($CDCl_3$): 1.23 (d, 6H), 1.50-2.0 (m, 2H), 2.21 (s, 1H), 2.42-2.74 (m, 2H), 3.08 (m,1H), 4.28 (m, 1H), 5.15 (m, 1H), 5.50 (dd,1H), 6.68 (d,1H), 7.00 (t, 2H), 7.31-7.58 (m,7H).

The product is a racemate that may be resolved to obtain the 4R,6S and 4S,6R enantiomers.

## EXAMPLE 12

Sodium erythro -(E)-3,5-dihydroxy-7-[3'-(4"-fluorophenyl)-5'-(1"-methylethyl)-1'-phenyl-1H-pyrazol-4'-yl]-hept-6-enoate (Reaction e)) (Compound no. 12 = Compound no. 9 but as erythro isomer).

The product may be obtained analogously to Example 7. It is a racemate that may be resolved to obtain the 3R,5S and 3S,5R enantiomers.

## EXAMPLE 13

Ethyl (±)-erythro -(E)-3,5-dihydroxy-7-[5'-(4"-fluorophenyl)-3'-(1"-methylethyl)-1'-phenyl-1H-pyrazol-4'-oyl]-hept-6-enoate (Compound no. 13 of formula IDa in ethyl ester form, erythro isomer) ($R_1$ = i-$C_3H_7$, $R_5$ =p-F, $R_2$ - $R_4$,$R_6$,$R_7$ = H, $R_{10}$ = H, X = (E)-CH=CH).

### Step 1(Reaction DA)

Ethyl 2-(4'-fluorobenzoxyl)-4-methyl-3-oxopentanoate (Compound LXXIIIa) (type LXXIII, $R_1$ = i-$C_3H_7$, $R_5$ = p-F, $R_6$,$R_7$ = H, $R_{15}$ = $C_2H_5$).

21.0 g (524 mmoles) of 60% sodium hydride/mineral oil is washed twice with hexane, the hexane is decanted, the sodium hydride is dried in a stream of nitrogen and suspended in 500 ml of dry tetrahydrofuran, the suspension is cooled to 0°, 50.0 g (238 mmoles) of Ethyl 3-(4'-fluorophenyl)-3-oxopropanoate is added dropwise over a period of 20 minutes at 0°, the reaction mixture is allowed to warm to 20°-25°, stirred at this temperature for 30 minutes and cooled to 0°. 38.0 g (357 mmoles) of isobutyryl chloride is added dropwise with stirring at 0°, and the reaction mixture is allowed to warm to 20°-25°, stirred at 20°-25° for 3 hours and cooled to 0°, the reaction mixture being stirred under nitrogen throughout. The reaction mixture is quenched at 0° with water, sufficient water being added to form a homogeneous mixture, the tetrafuran is evaporated at reduced pressure, and the aqueous solution is

acidified to pH 1 with 10% hydrochloric acid and extracted twice with 200 ml portions of diethyl ether. The diethyl ether extracts are combined, washed with saturated sodium chloride solution, dried over anhydrous magnesium sulfate, filtered and evaporated at reduced pressure to obtain the crude product as an orange oil.

Step 2(Reaction DB)

Ethyl 5-(4'-fluorophenyl)-3-(1'-methylethyl)-1-phenyl-1H-pyrazole-4-carboxylate (Compound LXXIVa) (type LXXIV; Py = PyD, $R_1$ = i-$C_3H_7$, $R_5$ = p-F, $R_2$ - $R_4$,$R_6$,$R_7$ = H, $R_{15}$ = $C_2H_5$).
38.6 g (357 mmoles) of phenylhydrazine is added portionwise to a solution of 72.99 g (≦ 238 mmoles) of crude Compound LXXIIIa in 300 ml of glacial acetic acid stirred at 20°-25° under nitrogen (the addition being slightly exothermic), and the reaction mixture is stirred at 20°-25° under nitrogen for 16 hours and poured into 700 ml of water. The mixture is extracted twice with 200 ml portions of diethyl ether, and the diethyl ether extracts are combined, extracted with saturated sodium bicarbonate solution (until the aqueous phase remains basic), washed with saturated sodium chloride solution, dried over anhydrous magnesium sulfate, filtered and evaporated at reduced pressure to obtain an orange gel. The gel is triturated with petroleum ether to obtain the product as a yellow powder , m.p. 91.5°-93°.

Step 3(Reaction DC)

5-(4'-fluorophenyl)-3-(1'-methylethyl)-1-phenyl-1H-pyrazole-4-methanol (Compound LXXVIa) (type LXXVI; Py = PyD, $R_1$ = i-$C_3H_7$, $R_5$ = p-F, $R_2$ - $R_4$,$R_6$,$R_7$ = H).
Analogous to Step 4 of Example 8.
The crude product is obtained as a tan powder.

Step 4(Reaction GA)

5-(4'-Fluorophenyl)-3-(1'-methylethyl)-1-phenyl-1H-pyrazole-4-carboxaldehyde (Compound CCIIa) (type CCII; Py = PyD, $R_1$ = i-$C_3H_7$, $R_5$ =p-F, $R_2$ - $R_4$,$R_6$,$R_7$ = H).
A solution of 33.95 g (≦ 109 mmoles) of crude Compound LXXVIa in 250 ml of methylene chloride is added rapidly dropwise to a solution of 70.75 g (328 mmoles) of pyridinium chlorochromate in 350 ml of methylene chloride stirred at 20°-25°, and the reaction mixture is stirred at 20°-25° for 4 hours 2.5. litres of diethyl ether is added, and the mixture is filtered through a 12.7 cm pad of silica gel and evaporated at reduced pressure to obtain a brown solid which is recrystallized from diethyl ether to obtain the product as a white solid. M.p. 110°-112°.

Step 5(Reaction GF)

Ethyl (E)-3-[5'-(4"-fluorophenyl)-3'-(1"-methylethyl)-1'-phenyl -1H-pyrazol-4'-yl]propenoate (Compound CCXIa] (type CCXI, Py = PyD, $R_1$ = i-$C_3H_7$, $R_5$ = p-F, $R_2$-$R_4$,$R_6$,$R_7$ = H, $R_{15}$ = $C_2H_5$).
761 mg (19 mmoles) of 60% sodium hydride/mineral oil is washed twice with hexane, the hexane is decanted, the sodium hydride is dried in a stream of nitrogen and suspended in 50 ml of dry tetrahydrofuran, the suspension is cooled to -15°, 4.06 g (18 mmoles) of triethyl phosphonoacetate is added dropwise with stirring at -15°, the reaction mixture is stirred at -15° for 45 minutes, a solution of 5.59 g (18 mmoles) of Compound CCIIa in 50 ml of dry tetryhydrofuran is added dropwise with stirring at -15°, and the reaction mixture is stirred at -15° for 45 minutes and allowed to warm to 20°-25°, the reaction mixture being stirred under nitrogen throughout. The reaction mixture is quenched at 20°-25° by the dropwise addition of saturated ammonium chloride solution, the tetrahydrofuran is evaporated at reduced pressure, and the solid residue is partitioned between ethyl acetate and water. The organic phase is separated, washed twice with saturated sodium chloride solution, dried over anhydrous magnesium sulfate, filtered and evaporated at reduced pressure to obtain the crude product as a yellow solid.

Step 6(Reaction GG)

(E)-3-[5'-(4"-Fluorophenyl]-3'-(1"-methylethyl)-1'-phenyl-1H-pyrazol-4'-yl]prop-2-en-1-ol (Compound CCXIIa) (type CCXII; Py = PyD, $R_1$ = i-$C_3H_7$, $R_5$ = p-F, $R_2$ - $R_4$,$R_6$,$R_7$ = H).
46.4 ml of 1.5M. diisobutylaluminium hydride/toluene (69.6 mmoles) is added dropwise to a solution of 6.59 g (≦ 17 mmoles) of crude Compound CCXIa in 150 ml of dry tetrahydrofuran stirred at 0° under nitrogen,

the reaction mixture is stirred at 0° under nitrogen for 45 minutes, quenched at 0° by the dropwise addition of saturated sodium sulfate solution and allowed to warm to 20°-25°, and sufficient 10% hydrochloric acid is added to dissolve the resulting gel. The organic phase is separated, the aqueous phase is extracted with diethyl ether, and the organic phase and diethyl ether extract are combined, washed with saturated sodium chloride solution, dried over anhydrous magnesium sulfate, filtered and evaporated at reduced pressure to obtain the crude product as a yellow foam.

**Step 7**(Reaction GH)

(E)-3-[5'-(4"-Fluorophenyl)-3'-(1"-methylethyl)-1'-phenyl-1H-pyrazol-4'-yl]propenal (Compound CCIVa) (type CCIV; Py = PyD, $R_1$ = i-$C_3H_7$, $R_5$ = p-F, $R_2$ - $R_4$,$R_6$,$R_7$ = H).

A mixture of 36.8 g (423 mmoles) activated maganese dioxide and a solution of 6.13 g (≦ 17 mmoles) of crude Compound CCXIIa in 150 ml of diethyl ether is stirred at 20°-25° for 16 hours and filtered through a 5.1 cm pad of Celite filter aid. The Celite is washed with ethyl acetate, and the ethyl acetate washing and the diethyl ether filtrate are combined and evaporated at reduced pressure to obtain a yellow solid which is recrystallized from diethyl ether to obtain the product as a yellow solid. M.p. 146°-150°.

**Step 8**

Ethyl (±)-(E)-7-[5'-(4"-fluorophenyl)-3'-(1"-methylethyl)-1'-phenyl-1H-pyrazol-4'-yl]-5-hydroxy-3-oxohept-6-enoate.

1.355 g (33.88 mmoles) of 60% sodium hydride/mineral oil is washed with hexane, the hexane is decanted, the sodium hydride is suspended in 100 ml of dry tetrahydrofuran, the suspension is cooled to -15°, 4.01 g (30.8 mmoles) of ethyl acetoacetate is added dropwise with stirring at -15°, the reaction mixture is stirred at -15° for 30 minutes, 20.2 ml of 1.6M. n-butyllithium/hexane (32.3 mmoles) added dropwise with stirring at -15°, the reaction mixture is stirred at -15° for 15 minutes, a solution of 5.14 g (15.4 mmoles) of Compound CCIVa in 50 ml of dry tetrahydrofuran is added dropwise with stirring at -15°, and the reaction mixture is stirred at -15° for 1 hour, the reaction mixture being stirred under nitrogen throughout. The reaction mixture is quenched at -15° with saturated ammonium chloride solution and warmed to 20°-25°, the tetrahydrofuran is evaporated at reduced pressure, and the residue is partitioned between diethyl ether and water. The aqueous phase is extracted with diethyl ether, and the diethyl ether extract and diethyl ether phase are combined, washed with saturated sodium chloride solution, dried over anhydrous magnesium sulfate, filtered and evaporated at reduced pressure to obtain a yellow foam which is chromatographed on a silica gel column (350 g) utilizing 1:1 diethxyl ether/hexane as the eluant to obtain the produot as a yellow oil.

The product is a racemate that may be resolved to obtain the R and S enantiomers.

**Step 9**(Reaction a))

Ethyl (±)-erythro -(E)-3,5-dihydroxy-7-[5'-(4"-fluorophenyl)-3'-(1"-methylethyl)-1'-phenyl-1H-pyrazol-4'-oyl]-hept-6-enoate (Compound no. 13).

23.34 ml of 1.0M. tri-n-butylborane/tetrhydrofuran (23.34 mmoles) is added via syringe to a solution of 5.42 g (11.67 mmoles) of the title compound of Step 8 in 100 ml of dry tetrahydrofuran stirred at 20°-25°, air is bubbled through the reaction mixture for 2 minutes with stirring, the reaction mixture is stirred at 20°-25° for 1 hour and cooled to -78°, 2.21 g (58.35 mmoles) of sodium borohydride is added, the reaction mixture is stirred at -55° for 16 hours and at 0° for 1 hour and, if thin layer chromatography reveals the presence of some starting material, the reaction mixture is cooled to -55°, an additional 1.1 g (29.1 mmoles) of sodium borohydride is added, and the reaction mixture is stirred at -55° for 16 hours and warmed to 0°, the reaction mixture being stirred under nitrogen throughout. The reaction mixture is quenched at 0° by the addition of 10% hydrochloric acid (until bubbling ceases), warmed to 20°-25° and partitioned between diethyl ether and water. The aqueous phase is extracted with diethyl ether, and the diethyl ether extract and the diethyl ether phase are combined, washed with saturated sodium chloride solution, dried over anhydrous magnesium sulfate, filtered and evaporated at reduced pressure to a clear oil. The oil is dissolved in 10 ml of isopropanol and the solution is evaporated at reduced prssure, this procedure is repeated to obtain a pale green wax, the wax is dissolved in a minimum amount of hot isopropanol, and the solution is allowed to cool to 20°-25° and stored at -30° for 64 hours. The resulting waxy solid is collected by filtration and vacuum dried to obtain a white powder (erythro :threo = ~9:1). The powder is recrystallized from isopropanol to obtain a waxy solid which is vacuum dried to obtain a white powder (erythro :threo =

~19:1). The white powder is warmed in methanol at 40°-60° for 2 minutes, the methanol is evaporated at reduced pressure, the residue is warmed in methanol at 40°-60° for 2 minutes and the methanol is evaporated at reduced pressure, and this procedure is repeated once more to obtain a white foam which is recrystallized from benzene/hexane to obtain the product as a white solid, m.p. 101°-104°.
The product is a mixture of the erythro and threo racemates wherein the ratio of the former to the latter is ~19:1 and which, if desired, may be separated by conventional means to obtain the pure erythro and threo racemates. The former may be resolved to obtain the 3R,5S and 3S,5R enantiomers, of which the former is preferred, and the latter may be resolved to obtain the 3R,5R and 3S,5S enantiomers. The use of a non-stereoselective reduction would afford a mixture of all four enantiomers wherein the ratio of the erythro isomers to the threo isomers ranges from 3:2 to 2:3.

## EXAMPLE 14

Sodium erythro -(±)-(E)-3,5-dihydroxy-7-[5'-(4"-fluorophenyl)-3'-(1"-methylethyl)-1 H phenyl-1-pyrazol-4'-yl]-hept-6-enoate (Reaction e)) (Compound no. 14 of formula IDa in sodium salt foam; erythro isomer) ($R_1$ = i-$C_3H_7$, $R_5$ = p-F, $R_2$ - $R_4$,$R_6$,$R_7$ = H, $R_{10}$ = H, X = (E)-CH=CH). 4.2 ml of 0.5N. sodium hydroxide solution (2.1 mmoles) is added to a solution of 1.02 g (2.2 mmoles) of Compound no. 13 in 25 ml of 95% ethanol, the mixture is stirred at 20°-25° under nitrogen for 5 hours, the ethanol is evaporated at reduced pressure, and the residue is partitioned between diethyl ether and water. The aqueous phase is washed twice with diethyl ether and lyophilized at -78° for 16 hours. The residue is warmed to 20°-25° and lyophilized at -78° for another 16 hours to obtain the product as a flocculant white solid, 205°-210° (dec.).
The product is a mixture of the erythro and threo racemates wherein the ratio of the former to the latter is ~19:1 and which, if desired, may be separated by conventional means to obtain the pure erythro and threo racemates. The former may be resolved to obtain the 3R,5S and 3S,5R enantiomers, of which the former is preferred, and the latter be resolved to obtain the 3R,5R and 3S,5S enantiomers. The use of a starting material obtained by utilizing a non-steroselective reduction in Step 10 of Example 13 would afford a mixture of all four enantiomers wherein theratio of the erythro isomes to the threo isomers ranges from 3:2 to 2:3.

## EXAMPLE 15

Ethyl (±)-erythro -(E)-3,5-dihydroxy-7-[5'-(3",5"-dimethylphenyl)-3'-(1"-methylethyl)-1'-phenyl-1H-pyrazol-4'-yl]hept-6-enoate (Compound no. 15 of Formula IDa in ethyl ester form; erythro isomer) ($R_1$ = i-$C_3H_7$, $R_5$ = m-$CH_3$, $R_6$ = m-$CH_3$, $R_2$ - $R_4$,$R_7$ = H, $R_{10}$ = H; X = (E)-CH = CH). The product may be obtained as a solid foam analogously to Steps 2 to 9 of Example 13 from ethyl 2-(3',5'-dimethylbenzoyl-4-methyl-3-oxopentanoate. Said compound is synthesized from ethyl-4-methyl-3-oxopentanoate and 3,5-dimethylbenzoyl chloride by Reaction DE carried out analogously to Step 1 of Example 13.

N.M.R. ($CDCl_3$): 1.28 (t (J=7.5 Hz), 3H), 1.4 (d (J=7.5 Hz), 6H), 1.68 (m,.2H), 2.25 (s, 6H), 2.46 (m, 2H), 2.98 (bs, 1H), 3.22 (m, 1H), 3.74 (d (J=2.5 Hz), 1H), 4.16 (q (J=7.5Hz), 2H), 4.22 (m, 1H), 4.39 (m, 1H), 5.69 (dd ($J_1$=17.5Hz., $J_2$=7.5 Hz), 1H), 6.38 (d (J=17.5Hz.), 1H), 6.79 (bs,2H), 6.95 (bs,1H), 7.2 (m,5H).

The product is a mixture of the erythro and threo racemates wherein the ratio of the former to the latter is ~9:1 and which, if desired, may be separated by conventional means to obtain the pure erythro and threo racemates. The former may be resolved to obtain the 3R,SS and 3S,5R enantiomers, of which the former is preferred, and the latter may be resolved to obtain the 3R,5R and 3S,5S enantiomers. The use of a non-stereoselective reduction would afford a mixture of all four enantiomers wherein the ratio of the erythro isomers to the threo isomers ranges from 3:2 to 2:3.

## EXAMPLE 16

Sodium (±)-erythro -(E)-3,5-dihydroxy-7-[5'-(3",5"-dimethylphenyl)-3'-(1"-methylethyl)-1'-phenyl-1H-pyrazol-4'-yl]hept-6-enoate (Compound no. 16 = Compound no. 15 but in Na salt rather than ethyl ester form). The product may be obtained from Compound no. 15 analgously to Example 14. M.p. 203°-210° (dec.).
The product is a mixture of the erythro and threo racemates wherein the ratio of the former to the latter is ~9:1 and which, if desired, may be separated by conventional means to obtain the pure erythro and threo racemates. The former may be resolved to obtain the 3R,5S and 3S,5R enantiomers, of which the former is preferred, and the latter may be resolved to obtain the 3R,5R and 3S,5S enantiomers. The use of a starting material prepared by the process of Example 15 but wherein the last step is a non-stereoselective reduction

would afford a mixture of all four enantiomers wherein the ratio of the erythro isomers to the threo isomers ranges from 3.:2 to 2:3.

The following compounds may be prepared analogously to the examples or as otherwise described hereinbefore.

- Ethyl (±)-erythro -(E)-3,5-dihydroxy-7-[1',5'-diphenyl-3'-(1"-methylethyl)-1H-pyrazol-4'-oyl]hept-6-enoate (Compound no. 17 of formula IDa in ethyl ester form, erythro isomer) ($R_1$ = i-$C_3H_7$, $R_2$ - $R_7$ = H, $R_{10}$ = H, X = (E)-CH=CH)
  N.M.R. ($CDCl_3$) : 1.26 (t,3H), 1.41 (d,6H), 1.72 (m,2H), 2.49(m,2H),2.96(bs, 1H),3.25(m,1H),3.69 (bs,1H), 4.18 (q, 2H), 4.25 (m,1H), 4.42 (m, 1H), 5.68 (dd, 1H), 6.4 (d, 1H), 7.24 (m,10H),
- Sodium (±)-erythro -(E)-3,5-dihydroxy-7-[1',5'-diphenyl-3'-(1"-methylethyl)-1H-pyrazol-4'-oyl]hept-6-enoate (Compound no. 18 (as compound no. 17) except in sodium salt rather than ethyl ester form), m.p. = 206-210° (dec.)
- Sodium (±)-erythro -3,5-dihydroxy-7-[5'-(4"-fluorophenyl)-3'-(1"-methylethyl)-1'-phenyl-1H-pyrazol-4'-oyl]heptanoate (Compound no. 19 of formula IDa in sodium salt form, erythro isomer) ($R_1$ = i-$C_3H_7$, $R_5$ = p-F, $R_2$ - $R_4$,$R_6$,$R_7$ = H, $R_{10}$ = H, X = $(CH_2)_2$), m.p. 198-210° (dec.),
  N.M.R. ($CD_3OD$): 1.36 (d,6H); 1.55 (m,4H), 2.3 (m,4H), 3.66 (m-1H), 4.08 (m,1H), 7.2 (m, 9H).

All nuclear magnetic resonance spectra were taken at ambient temperature on a 200 MHz. spectrometer. All chemical shifts are given in p.p.m. (δ) relative to tetramethylsilane, and where a single value is given for anything other than a sharp singlet, it is its centre point. In the N.M.R. data:

| | |
|---|---|
| bm = broad multiplet | bs = broad singlet |
| d = doublet | dd = doublet of a doublet |
| m = multiplet | q = quartet |
| s = singlet | t = triplet |

In the optical rotation data, the concentrations (c) are given in g/100 ml.

Throughout the examples the term "reduced pressure" denotes aspirator pressure. Where no solvent is specified in connection with a solution, the solvent is water and all solvent mixtures are by volume. When a reaction is carried out under nitrogen, dry nitrogen is used to maintain anhydrous conditions (except where the reaction medium contains water).

## Claims

1. A compound of formula I

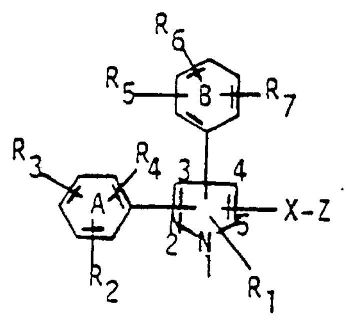

I

wherein $R_1$ is $C_{1-6}$alkyl,
each of $R_2$ and $R_5$ is independently hydrogen, $C_{1-4}$alkyl, $C_{1-4}$alkoxy (except t-butoxy), trifluoromethyl, fluoro, chloro, phenyl, phenoxy or benzyloxy,
each of $R_3$ and $R_6$ is independently hydrogen, $C_{1-3}$alkyl, $C_{1-3}$alkoxy, trifluoromethyl, fluoro, chloro, phenoxy or benzyloxy,
each of $R_4$ and $R_7$ is independently hydrogen, $C_{1-2}$alkyl, $C_{1-2}$alkoxy, fluoro or chloro,

with the proviso that there may only be a single trifluoromethyl, phenoxy or benzyloxy substituent in each of rings A and B,
X is $-(CH_2)_2-$ or $-CH=CH-$
and Z is

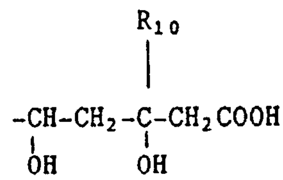

II

wherein $R_{10}$ is hydrogen or $C_{1-3}$alkyl,
whereby either the -X-Z group is in the 4-position of the pyrazole ring and $R_1$ is in the 3- or 5-position, or the -X-Z group is in the 5-position and $R_1$ is in the 1- or 4-position
in free acid form, or in the form of a physiologically acceptable ester thereof, or in the form of a δ-lactone thereof wherein Z has formula IIb,

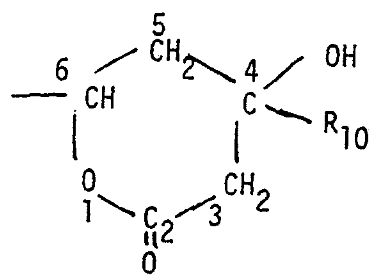

IIb

wherein $R_{10}$ is as defined above,
or in salt form.

2. A compound according to claim 1 wherein
$R_1$ is $C_{1-6}$alkyl not containing an asymmetric carbon atom,
each of $R_2$ and $R_5$ is independently hydrogen, $C_{1-3}$alkyl, n-butyl, i-butyl, t-butyl, $C_{1-3}$alkoxy, n-butoxy, sec-butoxy, trifluoromethyl, fluoro, chloro, phenyl, phenoxy or benzyloxy,
each of $R_3$ and $R_6$ is independently hydrogen, $C_{1-3}$alkyl, $C_{1-3}$alkoxy, trifluoromethyl, fluoro, chloro, phenoxy or benzyloxy,
each of $R_4$ and $R_7$ is independently hydrogen, $C_{1-2}$alkyl, $C_{1-2}$alkoxy, fluoro or chloro,
with the provisos that not more than one of $R_2$ and $R_3$ is trifluoromethyl, not more than one of $R_2$ and $R_3$ is phenoxy, not more than one of $R_2$ and $R_3$ is benzyloxy, not more than one of $R_5$ and $R_6$ is trifluoromethyl, not more than one of $R_5$ and $R_6$ is phenoxy, and not more than one of $R_5$ and $R_6$ is benzyloxy,
X is as defined in claim 1, and

Z is 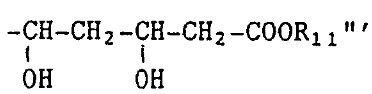

or

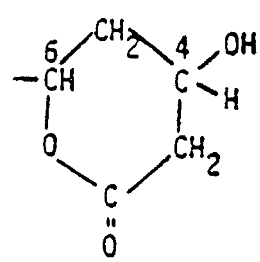

wherein $R_{11}'''$ is hydrogen, $R_{12}$ or M,

wherein $R_{12}$ is a physiologically acceptable and hydrolyzable ester group, and
M is a pharmaceutically acceptable cation.

**3.** A compound according to claim 1 selected from erythro -(E)-3R,5S-dihydroxy-7-[4'-(4"-fluorophenyl)-1'-(1"-methylethyl)-3'-phenyl-1H-pyrazol-5'-yl]hept-6-enoate and erythro -(±)-(E)-3,5-dihydroxy-7-[5'-(4"-fluorophenyl)-3'-(1"-methylethyl)-1'-phenyl-1-pyrazol-4'-yl]hept-6-enoate, in free acid or salt form.

**4.** A compound acocrding to claim 3 in sodium salt form.

**5.** A pharmaceutical composition comprising a compound according to claim 1 as appropriate in free acid form or in the form of a physiologically-hydrolysable and -acceptable ester or a δ-lactone thereof wherein Z has formula IIb as defined in claim 1, or in pharmaceutically acceptable salt form, together with a pharmaceutically acceptable diluent or carrier.

**6.** A compound according to claim 1 as appropriate in free acid form or in the form of a physiologically-hydrolysable and-acceptable ester or a δ-lactone thereof wherein Z has formula IIb as defined in claim 1, or in pharmaceutically acceptable salt form, for use as a pharmaceutical.

**7.** A compound according to claim 1 as appropriate in free acid form or in the form of a physiologically-hydrolysable and -acceptable ester or a δ-lactone thereof wherein Z has formula IIb as defined in claim 1, or in pharmaceutically acceptable salt form, for use in inhibiting cholesterol biosynthesis or treating atherosclerosis.

**8.** A process for preparing a compound according to claim 1 which comprises hydrolysing a compound of formula I in ester or δ-lactone form wherein Z has formula IIb as defined in claim 1, or esterifying or lactonising a compound of formula I in free acid form, and when a free carboxyl group is present recovering the compound obtained in free acid form or in the form of a salt.

**9.** A process for preparing a compound according to claim 1 wherein $R_{10}$ is hydrogen which comprises reducing a compound of formula XX

$$Py{-}X{-}\underset{OH}{\underset{|}{C}}H{-}CH_2{-}\underset{O}{\underset{||}{C}}{-}CH_2{-}COOR_{13} \qquad XX$$

wherein $R_{13}$ is a radical forming an ester
Py is a group of formula wherein $R_1$ to $R_7$ are as defined in claim

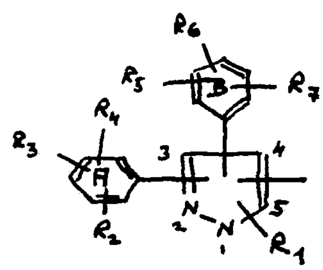

1, including provisos,
and X is as defined in claim 1,
and when a free carboxyl is present, recovering the compound obtained in free acid form or in the form of a salt.

**10.** A process for preparing a compound according to claim 1 wherein $R_{10}$ is $C_{1-3}$alkyl which comprises hydrolysing a compound of formula XVIIE

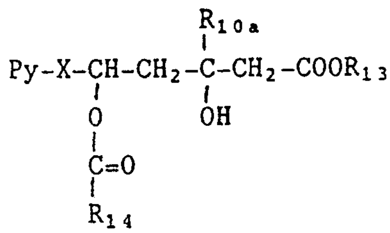

XVIIE

wherein $R_{10a}$ is $C_{1-3}$alkyl, $R_{14}$ is part of an ester forming group, $R_{13}$ and Py are as defined in claim 9 and, X is as defined in claim 1,
and when a free carboxyl is present, recovering the compound obtained in free acid form or in the form of a salt.

**11.** A process for preparing a compound according to claim 1 which comprises deprotecting a compound of formula V

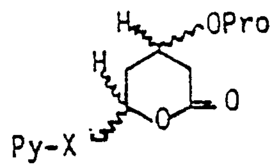

V

wherein X is as defined in claim 1, Py is as defined in claim 9 and Pro is a protecting group,
and when a free carboxyl is present, recovering the compound obtained in free acid form or in the form of a salt.

**12.** A process for preparing a compound according to claim 1 which comprises deprotecting a compound of formula XI

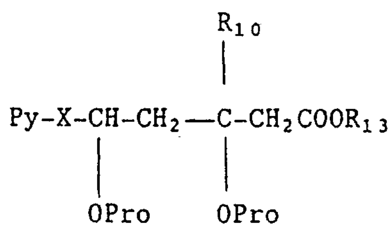

XI

wherein X and $R_{10}$ are as defined in claim 1, $R_{13}$ is as defined in claim 10, Pro is as defined in claim 11,
and Py is as defined in claim 9,
and when a free carboxyl is present, recovering the compound obtained in free acid form or in the form of a salt.

**13.** A process for preparing a compound according to claim 1 in free acid or salt form which comprises hydrolysing a compound of formula I in the form of an ester or a δ-lactone wherein Z has formula IIb as defined in claim 1,
and when a free carboxyl is present, recovering the compound obtained in free acid form or in the form of a salt.

**14.** A process for preparing a compound according to claim 1 in ester or δ-lactone form wherein Z has formula IIb as defined in claim 1, which comprises esterifying or lactonising a compound of formula I in free acid form.

**15.** A compound of formula V

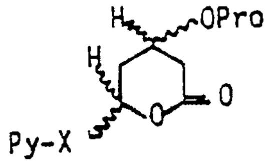

V

wherein X is as defined in claim 1, Py is as defined in claim 9 and Pro is a protecting group.

**16.** A compound of formula VII

$$PyCH_2CH_2 \sim Lac_3 \qquad VII$$

wherein Py is as defined in claim 9 and $Lac_3$ is

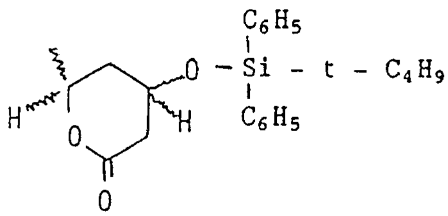

**17.** A compound of formula XI

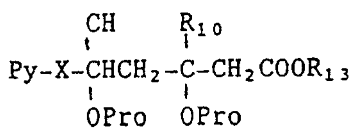

XI

wherein X and $R_{10}$ are as defined in claim 1, $R_{13}$ is as defined in claim 10, Pro is as defined in claim 15 and Py is as defined in claim 9.

**18.** A compound of formula XIII

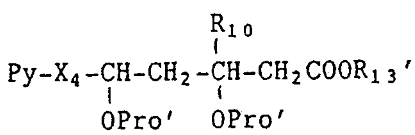

XIII

wherein Py is as defined in claim 9,
$X_4$ is $-(CH_2)_2-$,

Pro' is 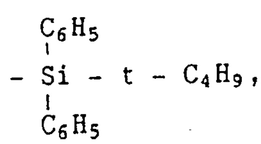

$R_{10}$ is as defined in claim 1 and
$R_{13}$' is $C_{1-3}$ alkyl, n-butyl, i-butyl, t-butyl or benzyl.

**19.** A compound of formula XVIIE

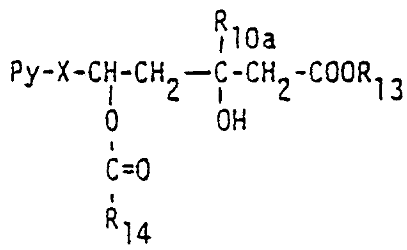 XVIIE

wherein Py and $R_{13}$ are as defined in claim 9,
X is as defined in claim 1, and
$R_{10}a$ and $R_{14}$ are as defined in claim 10.

**20.** A compound of formula XX

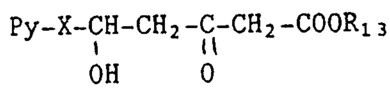 XX

wherein Py and $R_{13}$ are as defined in claim 9 and
X is as defined in claim 1.

**21.** A compound of formula CCIV

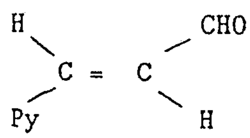 CCIV

wherein Py is as defined in claim 9.

Claims for the following Contracting State: AT

**1.** A process for the preparation of a compound of formula I

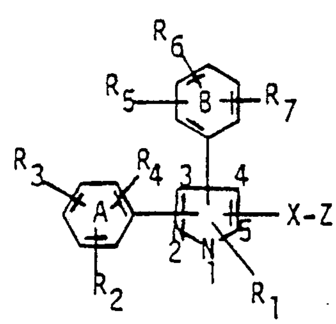 I

wherein $R_1$ is $C_{1-6}$alkyl,
each of $R_2$ and $R_5$ is independently hydrogen, $C_{1-4}$alkyl, $C_{1-4}$alkoxy (except t-butoxy), trifluoromethyl, fluoro, chloro, phenyl, phenoxy or benzyloxy,
each of $R_3$ and $R_6$ is independently hydrogen, $C_{1-3}$alkyl, $C_{1-3}$alkoxy, trifluoromethyl, fluoro, chloro, phenoxy or benzyloxy,
each of $R_4$ and $R_7$ is independently hydrogen, $C_{1-2}$alkyl, $C_{1-2}$alkoxy, fluoro or chloro,
with the proviso that there may only be a single trifluoromethyl, phenoxy or benzyloxy substituent in each of rings A and B,

X is $-(CH_2)_2-$ or -CH=CH-and Z is

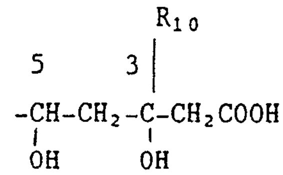 II

wherein $R_{10}$ is hydrogen or $C_{1-3}$alkyl,
whereby either the -X-Z group is in the 4-position of the pyrazole ring and $R_1$ is in the 3- or 5-position, or the -X-Z group is in the 5-position and $R_1$ is in the 1- or 4-position;
in free acid form, or in the form of a physiologically acceptable ester thereof, or in the form of a δ-lactone thereof wherein Z has formula IIb,

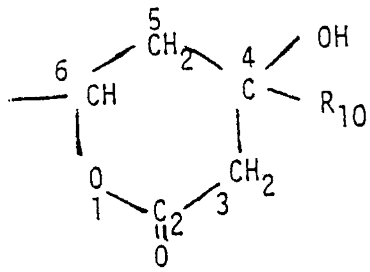 IIb

wherein $R_{10}$ is as defined above,
or in salt form,
which comprises hydrolysing a compound of formula I in ester or δ-lactone form wherein Z has formula IIb as defined above, or esterifying or lactonising a compound of formula I in free acid form, and when a free carboxyl group is present recovering the compound obtained in free acid form or in the form of a salt.

2. A process for preparing a compound of formula I according to claim 1 wherein $R_{10}$ is hydrogen which comprises reducing a compound of formula XX

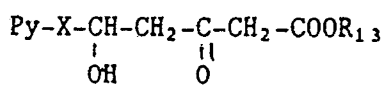 XX

wherein $R_{13}$ is a radical forming an ester
Py is a group of formula

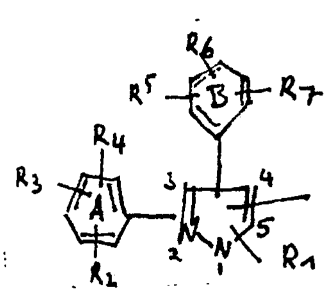

wherein $R_1$ to $R_7$ are as defined in claim 1, including the provisos
and X is as defined in claim 1,
and when a free carboxyl is present, recovering the compound obtained in free acid form or in the form of a salt.

**3.** A process for preparing a compound of formula I according to claim I wherein $R_{10}$ is $C_{1-3}$alkyl which comprises hydrolysing a compound of formula XVIIE

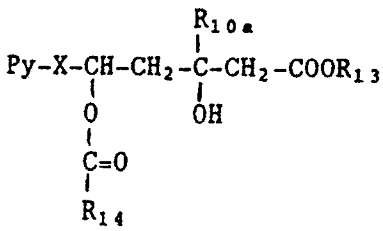

XVIIE

wherein $R_{10a}$ is $C_{1-3}$alkyl, $R_{14}$ is part of an ester forming group, X is as defined in claim 1;and $R_{13}$ and Py are as defined in claim 2, and when a free carboxyl is present, recovering the compound obtained in free acid form or in the form of a salt.

**4.** A process for preparing a compound of formula I according to claim 1 which comprises deprotecting a compound of formula V

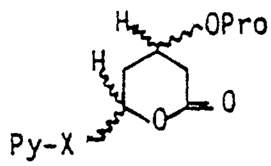

V

wherein X is as defined in claim and Py is as defined in claim 2 and Pro is a protecting group, and when a free carboxyl is present, recovering the compound obtained in free acid form or in the form of a salt.

**5.** A process for preparing a compound of formula I according to claim 1 which comprises deprotecting a compound of formula XI

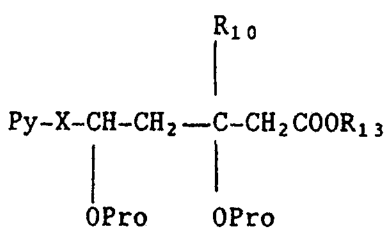

XI

wherein X and $R_{10}$ are as defined in claim 1, $R_{13}$ and Py are as defined in claim 2 and Pro is as defined in claim 3,
and when a free carboxyl is present, recovering the compound obtained in free acid form or in the form of a salt.

**6.** A process for preparing a compound of formula I according to claim 1 in free acid or salt form which comprises hydrolysing a compound of formula I in the form of an ester or a δ-lactone wherein Z has formula IIb as defined in claim 1,
and when a free carboxyl is present, recovering the compound obtained in free acid form or in the form of a salt.

**7.** A process for preparing a compound of formula I according to claim 1 in ester or δ-lactone form wherein Z has formula IIb as defined in claim 1, which comprises esterifying or lactonising a compound of formula I in free acid form.

**8.** A process according to any one of claim 1 to 7 for preparing a compound of formula I according to

claim 1 wherein

$R_1$ is $C_{1-6}$alkyl not containing an asymmetric carbon atom,

each of $R_2$ and $R_5$ is independently hydrogen, $C_{1-3}$alkyl, n-butyl, i-butyl, t-butyl, $C_{1-3}$alkoxy, n-butoxy, sec-butoxy, trifluoromethyl, fluoro, chloro, phenyl, phenoxy or benzyloxy,

each of $R_3$ and $R_6$ is independently hydrogen, $C_{1-3}$alkyl, $C_{1-3}$alkoxy, trifluoromethyl, fluoro, chloro, phenoxy or benzyloxy,

each of $R_4$ and $R_7$ is independently hydrogen, $C_{1-2}$alkyl, $C_{1-2}$alkoxy, fluoro or chloro,

with the provisos that not more than one of $R_2$ and $R_3$ is trifluoromethyl, not more than one of $R_2$ and $R_3$ is phenoxy, not more than one of $R_2$ and $R_3$ is benzyloxy, not more than one of $R_5$ and $R_6$ is trifluoromethyl, not more than one of $R_5$ and $R_6$ is phenoxy, and not more than one of $R_5$ and $R_6$ is benzyloxy,

X is as defined in claim 1, and

$$Z \text{ is } -\underset{\displaystyle OH}{\underset{|}{CH}}-CH_2-\underset{\displaystyle OH}{\underset{|}{CH}}-CH_2-COOR_{11}'''$$

or

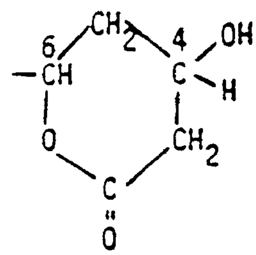

wherein $R_{11}'''$ is hydrogen, $R_{12}$ or M,

wherein $R_{12}$ is a physiologically acceptable and hydrolyzable ester group, and

M is a pharmaceutically acceptable cation.

**9.** A process according to any one of claims 1 to 7 for preparing a compound of formula I according to claim 1 selected from erythro -(E)-3R,5S-dihydroxy-7-[4'-(4"-fluorophenyl)-1'-(1"-methylethyl)-3'-phenyl-1H-pyrazol-5'-yl]hept-6-enoate and erythro -(±)-(E)-3,5-dihydroxy-7-[5'(4"-fluorophenyl)-3'-(1"-methylethyl)-1'-phenyl-1-pyrazol-4'-yl]hept-6-enoate in free acid or salt form.

**10.** A process according to any one of claims 1 to 7 for preparing a compound according to claim 9 in sodium salt form.

**11.** A process for preparing a pharmaceutical composition which comprises mixing together a compound of formula I according to claim 1 as appropriate in free acid form or in the form of a physiologically-hydrolysable and -acceptable ester or a δ-lactone thereof wherein Z has formula IIb as defined in claim 1, or in pharmaceutically acceptable salt form, and a pharmaceutically acceptable diluent or carrier.

## Revendications

**1.** Un composé de formule I

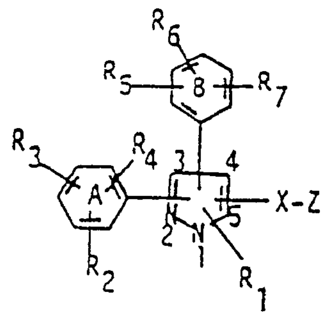

I

dans laquelle

$R_1$ signifie alkyle en $C_1$-$C_6$,

chacun de $R_2$ et $R_5$ signifie indépendamment l'hydrogène , alkyle en $C_1$-$C_4$, alcoxy en $C_1$-$C_4$ (excepté tert.-butoxy), trifluorométhyle, fluoro, chloro, phényle, phénoxy ou benzyloxy,

chacun de $R_3$ et $R_6$ signifie indépendamment l'hydrogène, alkyle en $C_1$-$C_3$, alcoxy en $C_1$-$C_3$, trifluorométhyle, fluoro, chloro, phénoxy ou benzyloxy,

chacun de $R_4$ et $R_7$ signifie indépendamment l'hydrogène, alkyle en $C_1$-$C_2$, alcoxy en $C_1$-$C_2$, fluoro ou chloro,

avec la condition qu'il n'y ait qu'un seul substituant trifluorométhyle, phénoxy ou benzyloxy dans chacun des cycles A et B,

X signifie $-(CH_2)_2-$ ou -CH = CH- et

Z signifie

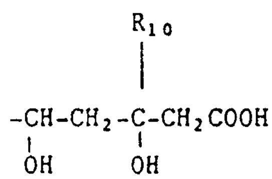

II

où $R_{10}$ signifie l'hydrogène ou alkyle en $C_1$-$C_3$,

et soit le groupe -X-Z est en position 4 du cycle pyrazole et $R_1$ est en position 3 ou 5, soit le groupe -X-Z est en position 5 et $R_1$ est en position 1 ou 4,

sous forme d'acide libre, sous forme d'un ester physiologiquement acceptable de ce composé, sous forme d'une δ-lactone de ce composé où Z correspond à la formule IIb

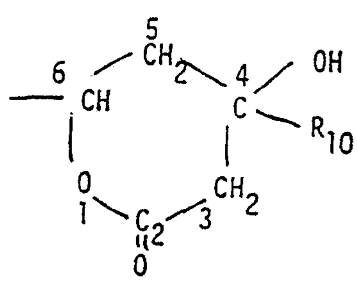

IIb

dans laquelle $R_{10}$ est tel que défini ci-dessus,

ou sous forme de sel.

**2.** Un composé selon la revendication 1, dans lequel

$R_1$ signifie alkyle en $C_1$-$C_6$ ne contenant pas d'atome de carbone asymétrique,

chacun de $R_2$ et $R_5$ signifie indépendamment l'hydrogène, alkyle en $C_1$-$C_3$, n-butyle, iso-butyle, tert.-butyle, alcoxy en $C_1$-$C_3$, n-butoxy, sec.-butoxy, trifluorométhyle, fluoro, chloro, phényle, phénoxy ou benzyloxy,

chacun de $R_3$ et $R_6$ signifie indépendamment l'hydrogène, alkyle en $C_1$-$C_3$, alcoxy en $C_1$-$C_3$,

trifluorométhyle, fluoro, chloro, phénoxy ou benzyloxy, chacun de $R_4$ et $R_7$ signifie indépendamment l'hydrogène, alkyle en $C_1$-$C_2$, alcoxy en $C_1$-$C_2$, fluoro ou chloro, avec les conditions que pas plus d'un symbole $R_2$ et $R_3$ signifie trifluorométhyle, pas plus d'un symbole $R_2$ et $R_3$ signifie phénoxy, pas plus d'un symbole $R_2$ et $R_3$ signifie benzyloxy, pas plus d'un symbole $R_5$ et $R_6$ signifie trifluorométhyle, pas plus d'un symbole $R_5$ et $R_6$ signifie phénoxy, et pas plus d'un symbole $R_5$ et $R_6$ signifie benzyloxy,
X est tel que défini à la revendication 1, et
Z signifie

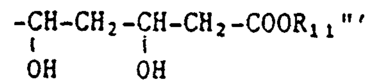

ou

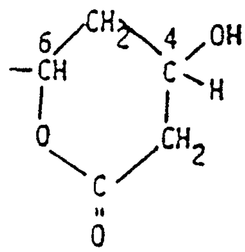

où $R_{11}'''$ signifie l'hydrogène, $R_{12}$ ou M,
$R_{12}$ signifiant un groupe ester physiologiquement acceptable et physiologiquement hydrolysable, et
M signifiant un cation pharmaceutiquement acceptable.

3. Un composé selon la revendication 1, choisi parmi l'érythro-(E)-3R,5S-dihydroxy-7-[4'-(4"-fluorophényl)-1'-(1"-méthyléthyl)-3'phényl-1H-pyrazole-5'-yl]hept-6-énoate et l'érythro-(±)-(E)-3,5-dihydroxy-7-[5'-(4"-fluorophényl)-3'-(1"-méthyléthyl)-1'-phényl-1-pyrazole-4'-yl] hept-6-énoate, sous forme d'acide libre ou de sel.

4. Un composé selon la revendication 3, sous forme de sel de sodium.

5. Une composition pharmaceutique comprenant un composé selon la revendication 1, comme cela convient sous forme d'acide libre, sous forme d'un ester physiologiquement hydrolysable et physiologiquement acceptable de ce composé, sous forme d'une δ-lactone de ce composé où Z correspond à la formule IIb telle que définie à la revendication 1, ou sous forme d'un sel pharmaceutiquement acceptable, ensemble avec un diluant ou véhicule pharmaceutiquement acceptable.

6. Un composé selon la revendication 1, comme cela convient sous forme d'acide libre, sous forme d'un ester physioloquement hydrolysable et physiologiquement acceptable de ce composé, sous forme d'une δ-lactone de ce composé où Z correspond à la formule IIb telle que définie à la revendication 1, ou sous forme d'un sel pharmaceutiquement acceptable, pour l'utilisation comme médicament.

7. Un composé selon la revendication 1, comme cela convient sous forme d'acide libre, sous forme d'un ester physiologiquement hydrolysable et physiologiquement acceptable de ce compose, sous forme d'une δ-lactone de ce composé où Z correspond à la formule IIb telle que définie à la revendication 1, ou sous forme d'un sel pharmaceutiquement acceptable, pour l'utilisation dans l'inhibition de la biosynthèse du cholestérol ou le traitement de l'athérosclérose.

8. Un procédé de préparation d'un composé selon la revendication 1, qui comprend l'hydrolyse d'un composé de formule I sous forme d'ester ou de δ-lactone où Z correspond à la formule IIb telle que définie à la revendication 1, ou l'estérification ou la lactonisation d'un composé de formule I sous forme d'acide libre, et, lorsqu'un groupe carboxy libre est présent, la récupération du composé obtenu sous forme d'acide libre ou sous forme d'un sel.

9. Un procédé de préparation d'un composé selon la revendication 1 dans lequel $R_{10}$ signifie l'hydrogène, qui comprend la réduction d'un composé de formule XX

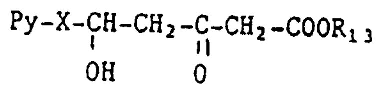

XX

dans laquelle
$R_{13}$ signifie un radical formant un ester,
$P_y$ signifie un groupe de formule

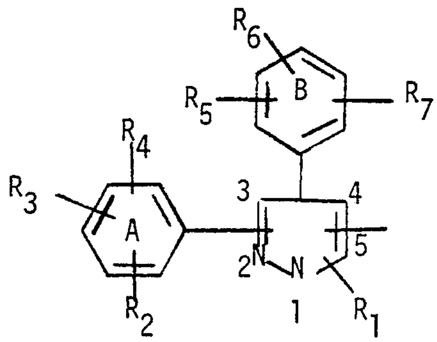

dans laquelle $R_1$ à $R_7$ sont tels que définis à la revendication 1, y compris les conditions, et
X est tel que défini à la revendication 1,
et, lorsqu'un groupe carboxy libre est présent, la récupération du composé obtenu sous forme d'acide libre ou sous forme d'un sel.

**10.** Un procédé de préparation d'un composé selon la revendication 1 dans lequel $R_{10}$ signifie alkyle en $C_1$-$C_3$, qui comprend l'hydrolyse d'un composé de formule XVIIE

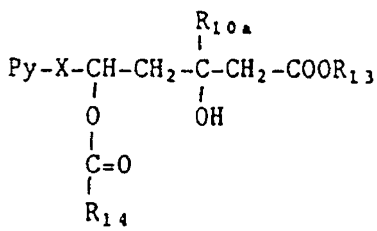

XVIIE

dans laquelle $R_{10a}$ signifie alkyle en $C_1$-$C_3$, $R_{14}$ est la partie d'un groupe formant un ester , $R_{13}$ et Py sont tels que définis à la revendication 9 et X est tel que défini à la revendication 1,
et, lorsqu'un groupe carboxy libre est présent, la récupération du composé obtenu sous forme d'acide libre ou sous forme d'un sel.

**11.** Un procédé de préparation d'un composé selon la revendication 1, qui comprend la déprotection d'un composé de formule V

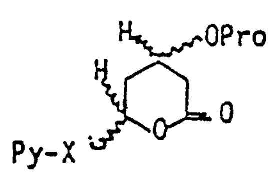

V

dans laquelle X est tel que défini à la revendication 1,
Py est tel que défini à la revendication 9 et Pro signifie un groupe protecteur,
et, lorsque un groupe carboxy libre est présent, la récupération du composé obtenu sous forme d'acide

libre ou sous forme d'un sel.

**12.** Un procédé de préparaiton d'un composé selon la revendication 1, qui comprend la déprotection d'un composé de formule XI

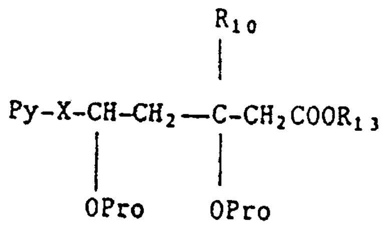

XI

dans laquelle X et $R_{10}$ sont tels que définis à la revendication 1, $R_{13}$ est tel que défini à la revendication 10, Pro est tel que défini à la revendication 11,
et Py est tel que défini à la revendication 9,
et, lorsqu'un groupe carboxy libre est présent, la récupération du composé obtenu sous forme d'acide libre ou sous forme d'un sel.

**13.** Un procédé de préparation d'un composé selon la revendication 1 sous forme d'acide libre ou sous forme d'un sel, qui comprend l'hydrolyse d'un composé de formule I sous forme d'un ester ou d'une δ-lactone où Z correspond à la formule IIb telle que définie à la revendication 1,
et, lorsqu'un groupe carboxy libre est présent, la récupération du composé obtenu sous forme d'acide libre ou sous forme d'un sel.

**14.** Un procédé de préparation d'un composé selon la revendication 1 sous forme d'ester ou de δ-lactone où Z correspond à la formule IIb telle que définie à la revendication 1, qui comprend l'estérification ou la lactonisation d'un composé de formule I sous forme d'acide libre.

**15.** Un composé de formule V

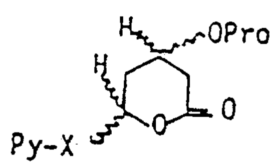

V

dans laquelle X est tel que défini à la revendication 1, Py est tel que défini à la revendication 9 et Pro signifie un groupe protecteur.

**16.** Un composé de formule VII

$$PyCH_2CH_2 \sim Lac_3 \qquad VII$$

dans laquelle Py est tel que défini à la revendication 9 et $Lac_3$ signifie

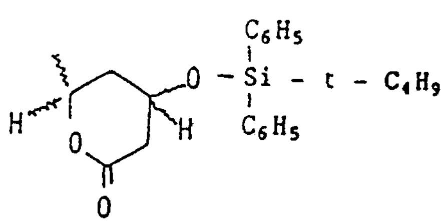

**17.** Un composé de formule XI

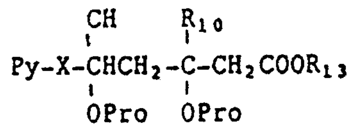

XI

dans laquelle X et $R_{10}$ sont tels que définis à la revendication 1, $R_{13}$ est tel que défini à la revendication 10, Pro est tel que défini à la revendication 15 et Py est tel que défini à la revendication 9.

**18.** Un composé de formule XIII

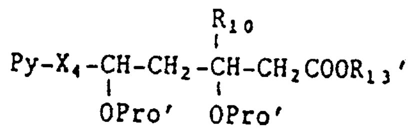

XIII

dans laquelle
Py est tel que défini à la revendication 9,
$X_4$ signifie $-(CH_2)_2$,
Pro' signifie

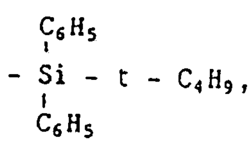

$R_{10}$ est tel que défini à la revendication 1, et
$R_{13}'$ signifie alkyle en $C_1$-$C_3$, n-butyle, iso-butyle, tert.-butyle ou benzyle.

**19.** Un composé de formule XVIIE

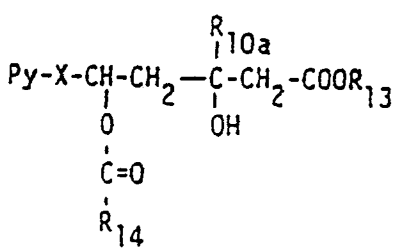

XVIIE

dans laquelle
Py et $R_{13}$ sont tels que définis à la revendication 9,
X est tel que défini à la revendication 1, et
$R_{10a}$ et $R_{14}$ sont tels que définis à la revendication 10.

**20.** Un composé de formule XX

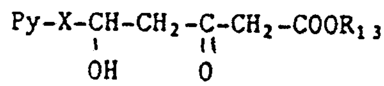 XX

dans laquelle
Py et $R_{13}$ sont tels que définis à la revendication 9, et
X est tel que défini à la revendication 1.

**21.** Un composé de formule CCIV

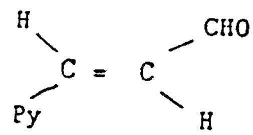 CCIV

dans laquelle Py est tel que défini à la revendication 9.

Revendications pour l'Etat contractant suivant: AT

**1.** Un procédé de préparation d'un composé de formule I

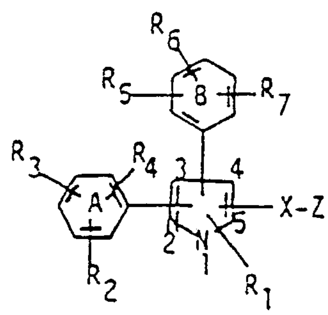 I

dans laquelle
$R_1$ signifie alkyle en $C_1$-$C_6$,
chacun de $R_2$ et $R_5$ signifie indépendamment l'hydrogène , alkyle en $C_1$-$C_4$, alcoxy en $C_1$-$C_4$ (excepté tert.-butoxy), trifluorométhyle, fluoro, chloro, phényle, phénoxy ou benzyloxy,
chacun de $R_3$ et $R_6$ signifie indépendamment l'hydrogène, alkyle en $C_1$-$C_3$, alcoxy en $C_1$-$C_3$, trifluorométhyle, fluoro, chloro, phénoxy ou benzyloxy,
chacun de $R_4$ et $R_7$ signifie indépendamment l'hydrogène, alkyle en $C_1$-$C_2$, alcoxy en $C_1$-$C_2$, fluoro ou chloro,
avec la condition qu'il n'y ait qu'un seul substituant trifluorométhyle, phénoxy ou benzyloxy dans chacun des cycles A et B,
X signifie $-(CH_2)_2-$ ou -CH = CH- et
Z signifie

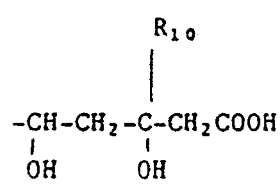 II

où $R_{10}$ signifie l'hydrogène ou alkyle en $C_1$-$C_3$,
et soit le groupe -X-Z est en position 4 du cycle pyrazole et $R_1$ est en position 3 ou 5, soit le groupe

-X-Z est en position 5 et $R_1$ est en position 1 ou 4, sous forme d'acide libre, sous forme d'un ester physiologiquement acceptable de ce composé, sous forme d'une δ-lactone de ce composé où Z correspond à la formule IIb

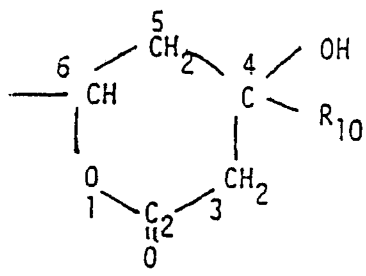

IIb

dans laquelle $R_{10}$ est tel que défini ci-dessus, ou sous forme de sel, qui comprend l'hydrolyse d'un composé de formule I sous forme d'ester ou de δ-lactone où Z correspond à la formule IIb telle que définie ci-dessus, ou l'estérification ou la lactonisation d'un composé de formule I sous forme d'acide libre, et, lorsqu'un groupe carboxy libre est présent, la récupération du composé obtenu sous forme d'acide libre ou sous forme d'un sel.

2. Un procédé de préparation d'un composé de formule I selon la revendication 1 dans lequel $R_{10}$ signifie l'hydrogène, qui comprend la réduction d'un composé de formule XX

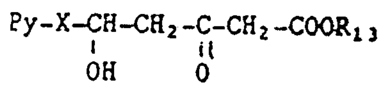

XX

dans laquelle
$R_{13}$ signifie un radical formant un ester,
Py signifie un groupe de formule

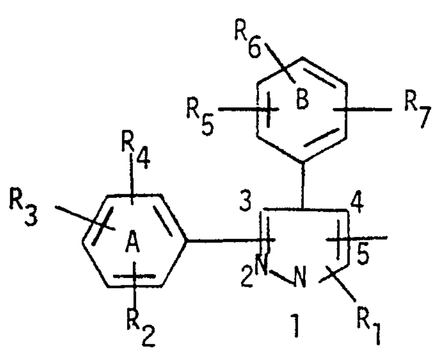

où $R_1$ à $R_7$ sont tels que définis à la revendication 1, y compris les conditions,
X est tel que défini à la revendication 1,
et, lorsqu' un groupe carboxy libre est présent, la récupération du composé obtenu sous forme d'acide libre ou sous forme d'un sel.

3. Un procédé de préparation d'un composé de formule I selon la revendication 1 dans lequel $R_{10}$ signifie alkyle en $C_1$-$C_3$, qui comprend l'hydrolyse d'un composé de formule XVIIE

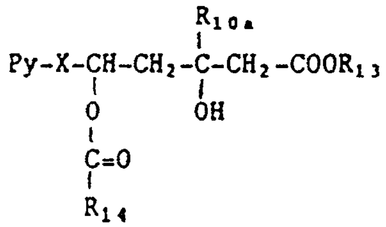

XVIIE

dans laquelle $R_{10a}$ signifie alkyle en $C_1$-$C_3$, $R_{14}$ est la partie d'un groupe formant un ester, X est tel que défini à la revendication 1; et $R_{13}$ et Py sont tels que définis à la revendication 2,
et, lorsqu'un groupe carboxy libre est présent, la récupération du composé obtenu sous forme d'acide libre ou sous forme d'un sel.

4. Un procédé de préparation d'un composé de formule I selon la revendication 1, qui comprend la déprotection d'un composé de formule V

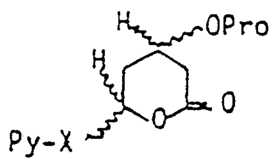

V

dans laquelle X est tel que défini à la revendication 1, Py est tel que défini à la revendication 2 et Pro signifie un groupe protecteur,
et, lorsqu'un groupe carboxy libre est présent, la récupération du composé obtenu sous forme d'acide libre ou sous forme d'un sel.

5. Un procédé de préparation d'un composé de formule I selon la revendication 1, qui comprend la déprotection d'un composé de formule XI

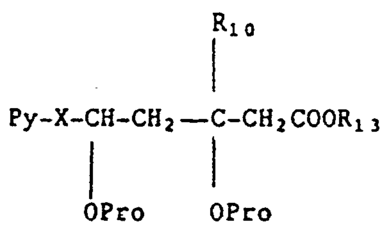

XI

dans laquelle X et $R_{10}$ sont tels que définis à la revendication 1, $R_{13}$ et Py sont tels que définis à la revendication 2 et Pro est tel que défini à la revendication 3,
et, lorsqu'un groupe carboxy libre est présent, la récupération du composé obtenu sous forme d'acide libre ou sous forme d'un sel.

6. Un procédé de préparation d'un composé de formule I selon la revendication 1 sous forme d'acide libre ou de sel, qui comprend l'hydrolyse d'un composé de formule I sous forme d'un ester ou d'une δ-lactone où Z correspond à la formule IIb telle que définie à la revendication 1, et, lorsqu'un groupe carboxy libre est présent, la récupération du composé obtenu sous forme d'acide libre ou sous forme d'un sel.

7. Un procédé de préparation d'un composé de formule I selon la revendication 1 sous forme d'ester ou de δ-lactone où Z correspond à la formule IIb telle que définie à la revendication 1, qui comprend l'estérification ou la lactonisation d'un composé de formule I sous forme d'acide libre.

8. Un procédé selon l'une quelconque des revendications 1 à 7, pour la préparation d'un composé de

formule I selon la revendication 1 dans lequel
$R_1$ signifie alkyle en $C_1$-$C_6$ ne contenant pas d'atome de carbone asymétrique,
chacun de $R_2$ et $R_5$ signifie indépendamment l'hydrogène, alkyle en $C_1$-$C_3$, n-butyle, iso-butyle, tert.-butyle, alcoxy en $C_1$-$C_3$, n-butoxy, sec.-butoxy, trifluorométhyle, fluoro, chloro, phényle, phénoxy ou benzyloxy,
chacun de $R_3$ et $R_6$ signifie indépendamment l'hydrogène, alkyle en $C_1$-$C_3$, alcoxy en $C_1$-$C_3$, trifluorométhyle, fluoro, chloro, phénoxy ou benzyloxy,
chacun de $R_1$ et $R_7$ signifie indépendamment l'hydrogène, alkyle en $C_1$-$C_1$, alcoxy en $C_1$-$C_2$, fluoro ou chloro, avec les conditions que pas plus d'un symbole $R_2$ et $R_3$ signifie trifluorométhyle, pas plus d'un symbole $R_2$ et $R_3$ signifie phénoxy, pas plus d'un symbole $R_2$ et $R_3$ signifie benzyloxy, pas plus d'un symbole $R_5$ et $R_6$ signifie trifluorométhyle, pas plus d'un symbole $R_5$ et $R_6$ signifie phénoxy, et pas plus d'un symbole $R_5$ et $R_6$ signifie benzyloxy,
X est tel que défini à la revendication 1, et
Z signifie

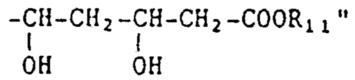

ou

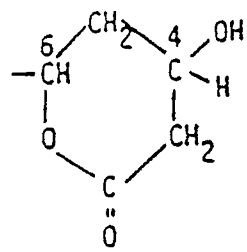

où $R_{11}'''$ signifie l'hydrogène, $R_{12}$ ou M,
$R_{12}$ signifiant un groupe ester physiologiquement acceptable et physiologiquement hydrolysable, et
M signifiant un cation pharmaceutiquement acceptable.

**9.** Un procédé selon l'une quelconque des revendications 1 à 7, pour la préparation d'un composé de formule I selon la revendication 1 choisi parmi l'érythro-(E)-3R,5S-dihydroxy-7-[4'-(4"-fluorophényl)-1'-(1"-méthyléthyl)-3'-phényl-1H-pyrazole-5'-yl]hept-6-énoate et 1'érythro-(±)-(E)-3,5-dihydroxy-7-[5'-(4"-fluorophényl)-3'-(1"-méthyléthyl)-1'-phényl-1-pyrazole-4'yl]hept-6-énoate, sous forme d'acide libre ou de sel.

**10.** Un procédé selon l'une quelconque des revendications 1 à 7, pour la préparation d'un composé selon la revendication 9 sous forme de sel de sodium.

**11.** Un procédé de préparation d'une composition pharmaceutique, qui comprend le mélange d'un composé de formule I selon la revendication 1, comme cela convient sous forme d'acide libre, sous forme d'un ester physiologiquement hydrolysable et physiologiquement acceptable de ce composé, sous forme d'une δ-lactone de ce composé où Z correspond à la formule IIb telle que définie à la revendication 1, ou sous forme d'un sel pharmaceutiquement acceptable, avec un diluant ou véhicule pharmaceutiquement acceptable.

## Ansprüche

**1.** Verbindung der Formel I

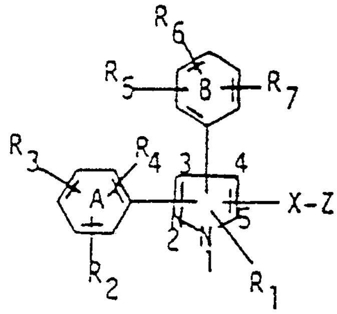

I

worin $R_1$ ein $C_1$-$C_6$-Alkylrest ist,
jeder der Reste $R_2$ und $R_5$ unabhängig Wasserstoff, ein $C_1$-$C_4$-Alkyl-, $C_1$-$C_4$-Alkoxy- (mit Ausnahme von t-Butoxy), Trifluormethyl-, Fluor-, Chlor-, Phenyl-, Phenoxy- oder Benzyloxyrest ist,
jeder der Reste $R_3$ und $R_6$ unabhängig Wasserstoff, ein $C_1$-$C_3$-Alkyl-, $C_1$-$C_3$-Alkoxy-, Trifluormethyl-, Fluor-, Chlor-, Phenoxy- oder Benzyloxyrest ist,
jeder der Reste $R_4$ und $R_7$ unabhängig Wasserstoff, ein $C_1$-$C_2$-Alkyl-, $C_1$-$C_2$-Alkoxyrest, Fluor oder Chlor ist,
mit dem Vorbehalt, daß nur ein einziger Trifluormethyl-, Phenoxy- oder Benzyloxysubstituent in jedem der Ringe A und B vorhanden sein kann,

X $-(CH_2)_2-$ oder $-CH{=}CH-$ ist

und Z $-CH(OH)-CH_2-C(R_{10})(OH)-CH_2COOH$ II

ist, worin $R_{10}$ Wasserstoff oder ein $C_1$-$C_3$-Alkylrest ist, wobei entweder sich die -X-Z-Gruppe in 4-Position des Pyrazolrings befindet und E in 3- oder 5-Position ist oder die -X-Z-Gruppe in 5-Position ist und $R_1$ in 1- oder 4-Position ist;
in Form der freien Säure oder in Form eines physiologisch annehmbaren Esters davon oder in Form eines $\delta$-Lactons davon, worin Z die Formel IIb

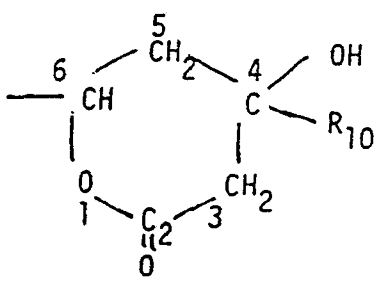

IIb

hat, worin $R_{10}$ wie oben definiert ist,
oder in Salzform.

2. Verbindung nach Anspruch 1, worin
$R_1$ ein $C_1$-$C_6$-Alkylrest ist, der kein asymmetrisches Kohlenstoffatom enthält,
jeder der Reste $R_2$ und $R_5$ unabhängig Wasserstoff, ein $C_1$-$C_3$-Alkyl-, n-Butyl-, i-Butyl-, t-Butyl-, $C_1$-$C_3$-Alkoxy-, n-Butoxy-, sec-Butoxy-, Trifluormethyl-, Fluor-, Chlor-, Phenyl-, Phenoxy- oder Benzyloxyrest ist,
jeder der Reste $R_3$ und $R_6$ unabhängig Wasserstoff, ein $C_1$-$C_3$-Alkyl-, $C_1$-$C_3$-Alkoxy-, Trifluormethyl-, Fluor-, Chlor-, Phenoxy- oder Benzyloxyrest ist,
jeder der Reste $R_4$ und $R_7$ unabhängig Wasserstoff, ein $C_1$-$C_2$-Alkyl-, $C_1$-$C_2$-Alkoxyrest, Fluor oder Chlor ist,
mit dem Vorbehalt, daß nicht mehr als einer der Reste $R_2$ und $R_3$ ein Trifluormethylrest ist, nicht mehr als einer der Reste $R_2$ und $R_3$ ein Phenoxyrest ist, nicht mehr als einer der Reste $R_2$ und $R_3$ ein

Benzyloxyrest ist, nicht mehr als einer der Reste $R_5$ und $R_6$ ein Trifluormethylrest ist, nicht mehr als einer der Reste $R_5$ und $R_6$ ein Phenoxyrest ist, und nicht mehr als einer der Reste $R_5$ und $R_6$ ein Benzyloxyrest ist,
X wie in Anspruch 1 definiert ist und

$$Z \quad -\underset{OH}{CH}-CH_2-\underset{OH}{CH}-CH_2-COOR_{11}''' \quad \text{oder}$$

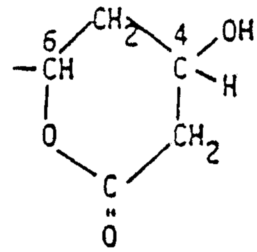

ist, worin $R_{11}'''$ Wasserstoff, $R_{12}$ oder M ist, worin $R_{12}$ eine physiologisch annehmbare und hydrolysierbare Estergruppe und M ein pharmazeutisch annehmbares Kation ist.

**3.** Verbindung nach Anspruch 1, ausgewählt aus erythro-(E)-3R,5S-Dihydroxy-7-[4'-(4"-fluorphenyl)-1'-(1"-methylethyl)-3'-phenyl-1H-pyrazol-5'-yl]hept-6-enoat und erythro-(±)-(E)-3,5-Dihydroxy-7-[5'-(4"-fluorphenyl)-3'-(1"-methylethyl)-1'-phenyl-1-pyrazol-4'-yl]hept-6-enoat, in Form der freien Säure oder in Salzform.

**4.** Verbindung nach Anspruch 3 in Form des Natriumsalzes.

**5.** Pharmazeutische Zusammensetzung, umfassend eine Verbindung nach Anspruch 1 je nachdem in Form der freien Säure oder in Form eines physiologisch hydrolysierbaren und annehmbaren Esters oder eines δ-Lactons davon, worin Z die Formel IIb,wie in Anspruch 1 definiert, hat oder in Form eines pharmazeutisch annehmbaren Salzes zusammen mit einem pharmazeutisch annehmbaren Verdünnungsmittel oder Träger.

**6.** Verbindung nach Anspruch 1, je nachdem in Form der freien Säure oder in Form eines physiologisch hydrolysierbaren und annehmbaren Esters oder eines δ-Lactons davon, worin Z die Formel IIb, wie in Anspruch 1 definiert, hat, oder in Form eines pharmazeutisch annehmbaren Salzes, zur Verwendung als Arzneimittel.

**7.** Verbindung nach Anspruch 1, je nachdem in Form der freien Säure oder in Form eines physiologisch hydrolysierbaren und annehmbaren Esters oder eines δ-Lactons davon, worin Z die Formel IIb, wie in Anspruch 1 definiert, hat oder in Form eines pharmazeutisch annehmbaren Salzes, zur Verwendung zur Hemmung der Cholesterinbiosynthese oder zur Behandlung von Atherosklerose.

**8.** Verfahren zur Herstellung einer Verbindung nach Anspruch 1, umfassend, daß man eine Verbindung der Formel I in Ester- oder δ-Lactonform, worin Z die Formel IIb, wie in Anspruch 1 definiert, hat, hydrolysiert oder eine Verbindung der Formel I in Form der freien Säure verestert oder lactonisiert und wenn eine freie Carboxylgruppe vorhanden ist, die Verbindung, die in Form der freien Säure oder in Form eines Salzes erhalten wird, gewinnt.

**9.** Verfahren zur Herstellung einer Verbindung nach Anspruch 1, worin $R_{10}$ Wasserstoff ist, umfassend, daß man eine Verbindung der Formel XX

$$Py-X-\underset{OH}{CH}-CH_2-\underset{O}{\overset{\|}{C}}-CH_2-COOR_{13} \qquad XX$$

worin $R_{13}$ ein einen Ester bildender Rest ist,
Py eine Gruppe der Formel

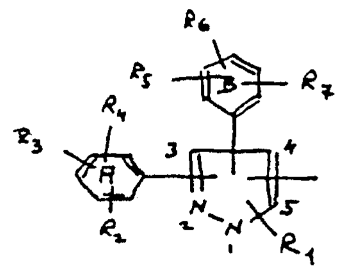

ist, worin $R_1$ bis $R_7$ wie in Anspruch 1 definiert sind einschließlich der Vorbehalte, und X wie in Anspruch 1 definiert ist, reduziert und wenn eine freie Carboxylgruppe vorhanden ist, die Verbindung, die in Form der freien Säure oder in Form eines Salzes erhalten wird, gewinnt.

**10.** Verfahren zur Herstellung einer Verbindung nach Anspruch 1, worin $R_{10}$ ein $C_1$-$C_3$-Alkylrest ist, umfassend, daß man eine Verbindung der Formel XVIIE

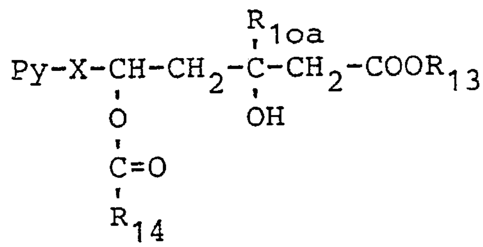 XVIIE

worin $R_{10a}$ ein $C_1$-$C_3$-Alkylrest ist, $R_{14}$ Teil einer esterbildenden Gruppe ist, $R_{13}$ und Py wie in Anspruch 9 definiert sind und X wie in Anspruch 1 definiert ist, hydrolysiert und wenn eine freie Carboxylgruppe vorhanden ist, die Verbindung, die in Form der freien Säure oder in Form eines Salzes erhalten wird, gewinnt.

**11.** Verfahren zur Herstellung einer Verbindung nach Anspruch 1, umfassend, daß man bei einer Verbindung der Formel V

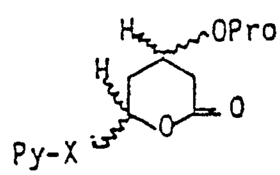 V

worin X wie in Anspruch 1 definiert ist, Py wie in Anspruch 9 definiert ist und Pro eine Schutzgruppe ist, die Schutzgruppe abspaltet und wenn eine freie Carboxylgruppe vorhanden ist, die Verbindung, die in Form der freien Säure oder in Salzform erhalten wird, gewinnt.

**12.** Verfahren zur Herstellung einer Verbindung nach Anspruch 1, umfassend,daß man bei einer Verbindung der Formel XI

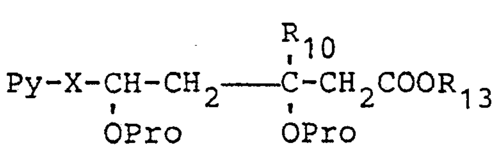 XI

worin X und $R_{10}$ wie in Anspruch 1 definiert sind, $R_{13}$ wie in Anspruch 10 definiert ist, Pro wie in Anspruch 11 definiert ist und Py wie in Anspruch 9 definiert ist, die Schutzgruppen abspaltet und,wenn eine freie Carboxylgruppe vorhanden ist, die Verbindung, die in

Form der freien Säure oder in Salzform erhalten wird, gewinnt.

**13.** Verfahren zur Herstellung einer Verbindung nach Anspruch 1 in Form der freien Säure oder in Salzform, umfassend, daß man eine Verbindung der Formel I in Form eines Esters oder eines δ-Lactons, worin Z die Formel IIb, wie in Anspruch 1 definiert, hat, hydrolysiert und,wenn eine freie Carboxylgruppe vorhanden ist, die Verbindung, die in Form der freien Säure oder in Salzform erhalten wird, gewinnt.

**14.** Verfahren zur Herstellung einer Verbindung nach Anspruch 1 in Ester- oder δ-Lactonform, worin Z die Formel IIb, wie in Anspruch 1 definiert, hat, umfassend, daß man eine Verbindung der Formel I in Form der freien Säure verestert oder lactonisiert.

**15.** Verbindung der Formel V

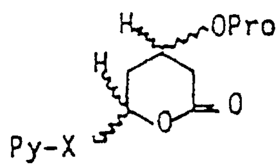

V

worin X wie in Anspruch 1 definiert ist, Py wie in Anspruch 9 definiert ist und Pro eine Schutzgruppe ist.

**16.** Verbindung der Formel VII

$$PyCH_2CH_2{\sim}Lac_3 \qquad VII$$

worin Py wie in Anspruch 9 definiert ist und $Lac_3$

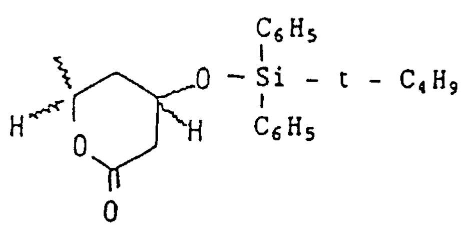

ist.

**17.** Verbindung der Formel XI

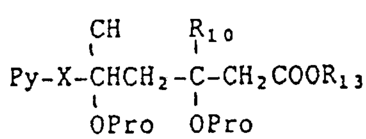

XI

worin X und $R_{10}$ wie in Anspruch 1 definiert sind, $R_{13}$ wie in Anspruch 10 definiert ist, Pro wie in Anspruch 15 definiert ist und Py wie in Anspruch 9 definiert ist.

**18.** Verbindung der Formel XIII

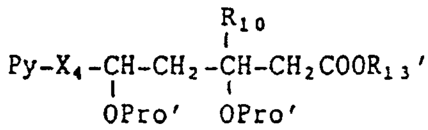 XIII

worin Py wie in Anspruch 9 definiert ist,
$X_4$ $-(CH_2)_2-$ ist,
Pro'

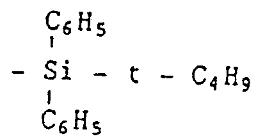

ist, $R_{10}$ wie in Anspruch 1 definiert ist und
$R_{13}$' ein $C_1$-$C_3$-Alkyl-, n-Butyl-, i-Butyl-, t-Butyl- oder Benzylrest ist.

**19.** Verbindung der Formel XVIIE

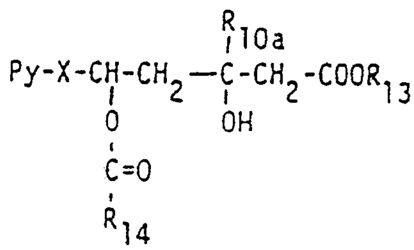 XVIIE

worin Py und $R_{13}$ wie in Anspruch 9 definiert sind,
X wie in Anspruch 1 definiert ist und
$R_{10a}$ und $R_{14}$ wie in Anspruch 10 definiert sind.

**20.** Verbindung der Formel XX

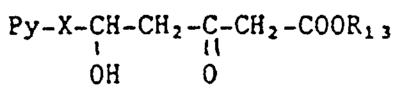 XX

worin Py und $R_{13}$ wie in Anspruch 9 definiert sind und X wie in Anspruch 1 definiert ist.

**21.** Verbindung der Formel CCIV

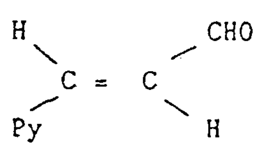 CCIV

worin Py wie in Anspruch 9 definiert ist.

Patentansprüche für folgenden Vertragsstaat: AT

**1.** Verfahren zur Herstellung einer Verbindung der Formel I

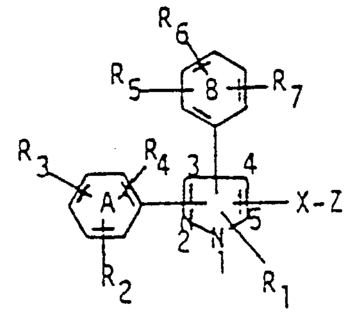

I

worin $R_1$ ein $C_1$-$C_6$-Alkylrest ist,
jeder der Reste $R_2$ und $R_5$ unabhängig Wasserstoff, ein $C_1$-$C_4$-Alkyl-, $C_1$-$C_4$-Alkoxy- (mit Ausnahme von t-Butoxy), Trifluormethyl-, Fluor-, Chlor-, Phenyl-, Phenoxy- oder Benzyloxyrest ist,
jeder der Reste $R_3$ und $R_6$ unabhängig Wasserstoff, ein $C_1$-$C_3$-Alkyl-, $C_1$-$C_3$-Alkoxy-, Trifluormethyl-, Fluor-, Chlor-, Phenoxy- oder Benzyloxyrest ist,
jeder der Reste $R_4$ und $R_7$ unabhängig Wasserstoff, ein $C_1$-$C_2$-Alkyl-, $C_1$-$C_2$-Alkoxyrest, Fluor oder Chlor ist,
mit dem Vorbehalt, daß nur ein einziger Trifluormethyl-, Phenoxy- oder Benzyloxysubstituent in jedem der Ringe A und B vorhanden sein kann,

X $-(CH_2)_2$ oder $-CH=CH-$ ist

und Z $-\underset{OH}{CH}-CH_2-\overset{R_{10}}{\underset{OH}{C}}-CH_2COOH$ II

ist, worin $R_{10}$ Wasserstoff oder ein $C_1$-$C_3$-Alkylrest ist, wobei entweder sich die -X-Z-Gruppe in 4-Position des Pyrazolrings befindet und E in 3- oder 5-Position ist oder die -X-Z-Gruppe in 5-Position ist und $R_1$ in 1- oder 4-Position ist;
in Form der freien Säure oder in Form eines physiologisch annehmbaren Esters davon oder in Form eines $\delta$-Lactons davon, worin Z die Formel IIb

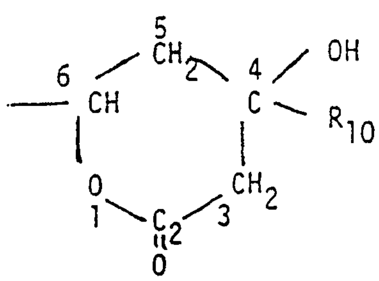

IIb

hat, worin $R_{10}$ wie oben definiert ist,
oder in Salzform,
umfassend, daß man eine Verbindung der Formel I in Ester- oder $\delta$-Lactonform, worin Z die Formel IIb, wie oben definiert, hat, hydrolysiert oder eine Verbindung der Formel I in Form der freien Säure verestert oder lactonisiert und, wenn eine freie Carboxylgruppe vorhanden ist, die Verbindung, die in Form der freien Säure oder in Form eines Salzes erhalten wird, gewinnt.

2. Verfahren zur Herstellung einer Verbindung der Formel I nach Anspruch 1, worin $R_{10}$ Wasserstoff ist, umfassend, daß man eine Verbindung der Formel XX

$Py-X-\underset{OH}{CH}-CH_2-\underset{\overset{\|}{O}}{C}-CH_2-COOR_{13}$ XX

worin $R_{13}$ ein einen Ester bildender Rest ist,
Py eine Gruppe der Formel

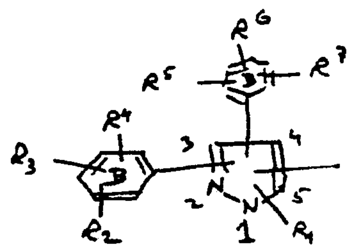

ist, worin $R_1$ bis $R_7$ wie in Anspruch 1 definiert sind, einschließlich der Vorbehalte, und X wie in Anspruch 1 definiert ist, reduziert und wenn eine freie Carboxylgruppe vorhanden ist, die Verbindung, die in Form der freien Säure oder in Salzform erhalten wird, gewinnt.

**3.** Verfahren zur Herstellung einer Verbindung der Formel I nach Anspruch 1, worin $R_{10}$ ein $C_1$-$C_3$-Alkylrest ist, umfassend, daß man eine Verbindung der Formel XVIIE

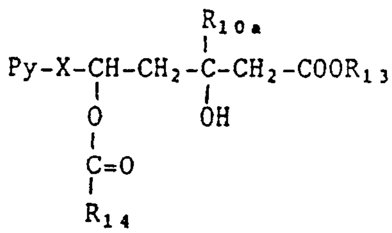

XVIIE

worin $R_{10a}$ ein $C_1$-$C_3$-Alkylrest ist, $R_{14}$ Teil einer esterbildenden Gruppe ist, X wie in Anspruch 1 definiert ist und $R_{13}$ und Py wie in Anspruch 2 definiert sind, hydrolysiert und, wenn eine freie Carboxylgruppe vorhanden ist, die Verbindung, die in Form der freien Säure oder in Salzform erhalten wird, gewinnt.

**4.** Verfahren zur Herstellung einer Verbindung der Formel I nach Anspruch 1, umfassend, daß man bei einer Verbindung der Formel V

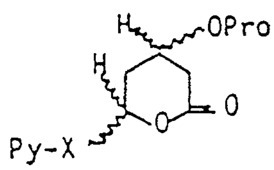

V

worin X wie in Anspruch 1 definiert ist und Py wie in Anspruch 2 definiert ist und Pro eine Schutzgruppe ist, die Schutzgruppe abspaltet und, wenn eine freie Carboxylgruppe vorhanden ist, die Verbindung, die in Form der freien Säure oder in Salzform erhalten wird, gewinnt.

**5.** Verfahren zur Herstellung einer Verbindung der Formel I nach Anspruch 1, umfassend, daß man bei einer Verbindung der Formel XI

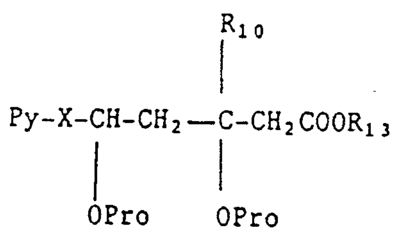

XI

worin X und $R_{10}$ wie in Anspruch 1 definiert sind, $R_{13}$ und Py wie in Anspruch 2 definiert sind und Pro wie in Anspruch 4 definiert ist, die Schutzgruppen abspaltet und, wenn eine freie Carboxylgruppe vorhanden ist, die Verbindung, die in Form der freien Säure oder in Salzform erhalten wird, gewinnt.

6. Verfahren zur Herstellung einer Verbindung der Formel I nach Anspruch 1 in Form der freien Säure oder in Salzform, umfassend, daß man eine Verbindung der Formel I in Esterform oder in Form eines $\delta$-Lactons, worin Z die Formel IIb, wie in Anspruch 1 definiert, hat, hydrolysiert und, wenn eine freie Carboxylgruppe vorhanden ist, die Verbindung, die in Form der freien Säure oder in Salzform erhalten wird, gewinnt.

7. Verfahren zur Herstellung einer Verbindung der Formel I nach Anspruch 1 in Ester- oder $\delta$-Lactonform, worin Z die Formel IIb, wie in Anspruch 1 definiert, hat, umfassend, daß man eine Verbindung der Formel I in Form der freien Säure verestert oder lactonisiert.

8. Verfahren nach einem der Ansprüche 1 bis 7 zur Herstellung einer Verbindung der Formel I nach Anspruch 1, worin
$R_1$ ein $C_1$-$C_6$-Alkylrest ist, der kein asymmetrisches Kohlenstoffatom enthält,
jeder der Reste $R_2$ und $R_5$ unabhängig Wasserstoff, eine $C_1$-$C_3$-Alkyl-, n-Butyl-, i-Butyl-, t-Butyl-, $C_1$-$C_3$-Alkoxy-, n-Butoxy-, sec-Butoxy-, Trifluormethyl-, Fluor-, Chlor-, Phenyl-, Phenoxy- oder Benzyloxygruppe ist,
jeder der Reste $R_3$ und $R_6$ unabhängig Wasserstoff, ein $C_1$-$C_3$-Alkyl-, $C_1$-$C_3$-Alkoxy-, Trifluormethyl-, Fluor-, Chlor-, Phenoxy- oder Benzyloxyrest ist,
jeder der Reste $R_4$ und $R_7$ unabhängig Wasserstoff, ein $C_1$-$C_2$-Alkyl-, $C_1$-$C_2$-Alkoxyrest, Fluor oder Chlor ist,
mit dem Vorbehalt, daß nicht mehr als einer der Reste $R_2$ und $R_3$ ein Trifluormethylrest ist, nicht mehr als einer der Reste $R_2$ und $R_3$ ein Phenoxyrest ist, nicht mehr als einer der Reste $R_2$ und $R_3$ ein Benzyloxyrest ist, nicht mehr als einer der Reste $R_5$ und $R_6$ ein Trifluormethylrest ist, nicht mehr als einer der Reste $R_5$ und $R_6$ ein Phenoxyrest ist und nicht mehr als einer der Reste $R_5$ und $R_6$ ein Benzyloxyrest ist,
X wie in Anspruch 1 definiert ist und

Z 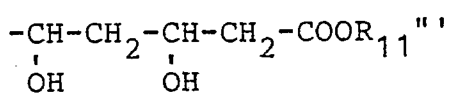 oder

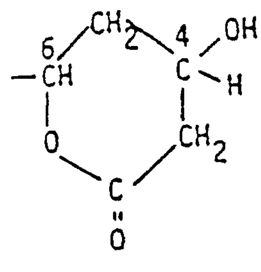

ist, worin $R_{11}'''$ Wasserstoff, $R_{12}$ oder M ist, worin $R_{12}$ eine physiologisch annehmbare und hydrolysierbare Estergruppe und M ein pharmazeutisch annehmbares Kation ist.

9. Verfahren nach einem der Ansprüche 1 bis 7 zur Herstellung einer Verbindung der Formel I nach Anspruch 1, ausgewählt aus erythro-(E)-3R,5S-Dihydroxy-7-[4'-(4"-fluorphenyl)-1'-(1"-methylethyl)-3'-phenyl-1H-pyrazol-5'-yl]hept-6-enoat und erythro-(±)-(E)-3,5-Dihydroxy-7-[5'-(4"-fluorphenyl)-3'-(1"-methylethyl-)1'-phenyl-1-pyrazol-4'-yl]hept-6-enoat, in Form der freien Säure oder in Salzform.

10. Verfahren nach einem der Ansprüche 1 bis 7 zur Herstellung einer Verbindung nach Anspruch 9 in Form des Natriumsalzes.

11. Verfahren zur Herstellung einer pharmazeutischen Zusammensetzung, umfassend, daß man eine Verbindung der Formel I nach Anspruch 1 je nachdem in Form der freien Säure oder in Form eines physiologisch hydrolysierbaren und annehmbaren Esters oder eines $\delta$-Lactons davon, worin Z die Formel IIb, wie in Anspruch 1 definiert, hat, oder in Form eines pharmazeutisch annehmbaren Salzes mit einem pharmazeutisch annehmbaren Verdünnungsmittel oder Träger vermischt.